# EUROPEAN PATENT APPLICATION

(11) **EP 3 047 856 A1**
(43) Date of publication of application: **27.07.2016**
(21) Application number: 15152415.4
(22) Date of filing: 23.01.2015
(51) Int. Cl.: A61K 39/245, C07K 14/005, C07K 16/08, A61K 39/00

(54) **CMV ANTIGENS AND USES THEREOF**

(71) Applicant: Novartis AG, 4056 Basel (CH)
(72) Inventor: Merola, Marcello, 53100 Siena (IT); Cortese, Mirko, 53100 Siena (IT); Calo, Stefano, 53100 Siena (IT); Carfi, Andrea, Cambridge, MA 02139 (US); Bruno, Luca, 53100 Siena (IT)
(74) Representative: Courgeon, Antoine

(57) **Abstract**

The invention relates to a recombinant human cytomegalovirus (CMV) protein dimeric complex, comprising CMV gH protein or a complex-forming fragment thereof, and CMV UL116 or a complex-forming fragment thereof. Also provided herein are nucleic acids encoding said gH/UL116 dimeric complex, host cells for recombinant expression of said gH/UL116 dimeric complex, and the use of said gH/UL116 dimeric complex for use as a vaccine antigen.

## Description

### FIELD OF THE INVENTION

This invention relates to cytomegalovirus (CMV) antigens suitable for vaccine uses.

### BACKGROUND

Human cytomegalovirus (HCMV) causes widespread, persistent human infections that vary with the age and immunocompetence of the host. It can remain latent throughout the lifetime of the host with sporadic reactivation events. The primary infection of hosts with a functional immune system is associated with mild symptoms although it may progress with fever, hepatitis, splenomegaly and a mononucleosis-like disease. In contrast, when primary infection or reactivation occurs in immunocompromised or immunodeficient hosts, they often experience life-threatening diseases, including pneumonia, hepatitis, retinitis and encephalitis (Sinclair and Sissons, J. Gen. Virol. 87:1763-1779, 2006). HCMV infection has been recognized for its association with three different populations: neonates with immature immune systems; transplant recipients with impaired immune systems due to the use of drugs and HIV-infected patients with compromised immune systems due to the decline of CD4⁺ T cells.

HCMV can be particularly devastating in neonates, causing defects in neurological development. In the industrialized countries, intrauterine viral infection is most common. Estimates suggest that between 0.6% and 0.7% (depending on the seroprevalence of the population examined) of all new neonates are infected in utero (Dollard et al., Rev. Med. Virol., 17(5):355-363, 2007). In the United States alone, this corresponds to approximately 40,000 new infections each year. Around 1.4% of intrauterine CMV infections occur from transmission by women with established infection. New maternal infection occurs in 0.7 to 4.1% of pregnancies and is transmitted to the fetus in about 32% of cases. Around 90% of infected infants are asymptomatic at birth and most will develop serious consequences of the infection over the course of several years, including mental retardation and hearing loss. Other infected children show symptomatic HCMV disease with symptoms of irreversible central nervous system involvement in the form of microencephaly, encephalitis, seizures, deafness, upper-motor neuron disorders and psychomotor retardation (Kenneson et al., Rev. Med. Virol., 17(4):253-276, 2007). In sum, approximately 8,000 children in the United States develop virus-related neurological disease each year. Congenital infection is the major driving force behind efforts to develop an HCMV vaccine.

Efforts to develop a HCMV vaccine began more than 40 years ago. Over the years a number of HCMV vaccines have been evaluated, including a whole virus vaccine, chimeric vaccines and subunit vaccines. The whole virus vaccine neither prevented infection or vial reactivation in immunized adult women, nor increased protection against diseases compared to seropositive individuals (Arvin et al., Clin. Infect. Dis. 39(2), 233-239, 2004). Each of the chimeric vaccines were well tolerated, but concerns about the potential risk of establishing a latent infection hindered the progression of those vaccines. The subunit vaccine approach, based on the assumption that immunity directed toward a limited number of dominant antigens, has showed low efficacy thus far. These results suggest that an effective vaccine may need to be directed towards multiple antigens expressed at different stages of viral replication.

CMV envelope glycoproteins gB, gH, gL, gM, gN, UL128, UL130, and UL131 represent attractive vaccine candidates as they are expressed on the viral surface and can elicit protective virus-neutralizing humoral immune responses. However, a need exists for developing alternative CMV antigens suitable for immunization.

### SUMMARY OF THE INVENTION

As disclosed and exemplified herein, the inventors discovered a novel human cytomegalovirus (CMV) protein dimeric complex, formed by CMV proteins gH and UL116.

Accordingly, the invention relates to a recombinant human cytomegalovirus (CMV) protein dimeric complex, comprising CMV gH protein or a complex-forming fragment thereof, and CMV UL116 or a complex-forming fragment thereof.

Also provided herein are immunogenic compositions comprising gH/UL116 dimeric complex. The immunogenic complex may further comprise an additional CMV protein or CMV protein complex. Suitable CMV antigens that can be combined with gH/UL116 dimeric complex include, e.g.: gB, gH, gL, gO, gM, gN; UL128, UL130, UL131, RL10, RL11, RL12, RL13, UL4, UL5, UL10, UL80.5, UL119, UL122, UL133, UL138, UL148A, UL1, UL7, UL9, UL16, UL18, UL20, UL40, UL41A, UL42, UL47, UL111A, UL124, UL132, UL136, UL141, an immunogenic fragment thereof, or a combination thereof. Suitable CMV protein complexes that can be combined with gH/UL116 dimeric complex include, e.g., gH/gL/UL128/UL130/UL131 pentameric complex, gH/gL complex, gH/gL/gO trimeric complex, or a combination thereof.

Also provided herein are nucleic acids encoding CMV gH/UL116 dimeric complex, as described herein. The nucleic acid can be a single nucleic acid construct (e.g., a single vector) encoding both gH and UL116. The nucleic acid can also be a combination of nucleic acid constructs (e.g., a first vector encoding gH and a second vector encoding UL116). The nucleic acid(s) may be used as a nucleic acid-based vaccine (e.g., a self-replicating RNA molecule encoding the gH/UL116 dimeric complex). The nucleic acid may also be used for recombinant production of the gH/UL116 dimeric complex described herein.

The invention also provides a host cell comprising the nucleic acids described herein. The host cells can be used to recombinantly express gH/UL116 dimeric complex. Preferably, the gH/UL116 dimeric complex can be secreted from the host cell. Preferred host cells are mammalian host cells, such as CHO cells or HEK-293 cells.

The invention also provides a cell culture comprising the host cell described herein. Preferably, the culture is at least 20 liter in size, and/or the yield of the gH/UL116 dimeric complex is at least 0.1 g/L.

The invention also provides a method of inducing an immune response against cytomegalovirus (CMV), comprising administering to a subject in need thereof an immunologically effective amount of the gH/UL116 dimeric complex described herein. The invention also provides a method of inhibiting cytomegalovirus (CMV) entry into a cell, comprising contacting the cell with the gH/UL116 dimeric complex described herein.

Also provided are use of the compositions described herein for inducing an immune response against cytomegalovirus (CMV), and use of the compositions described herein in the manufacture of a medicament for inducing an immune response against cytomegalovirus (CMV).

The invention further relates to a method of forming gH/UL116 dimeric complex described herein, comprising delivering nucleic acid(s) encoding gH and UL116 (or complex-forming fragment thereof) to a cell, and maintaining the cell under conditions suitable for expression of said gH and UL116 (or complex-forming fragment thereof), wherein the gH/UL116 dimeric complex is formed. The cell can be in vivo or in vitro. For in vitro purposes, any suitable host cell (e.g., a bacterial host or a eukaryotic cell line) can be used. For in vivo purposes, the cell can be an epithelial cell, an endothelial cell, or a fibroblast.

### BRIEF DESCRIPTION OF THE DRAWINGS

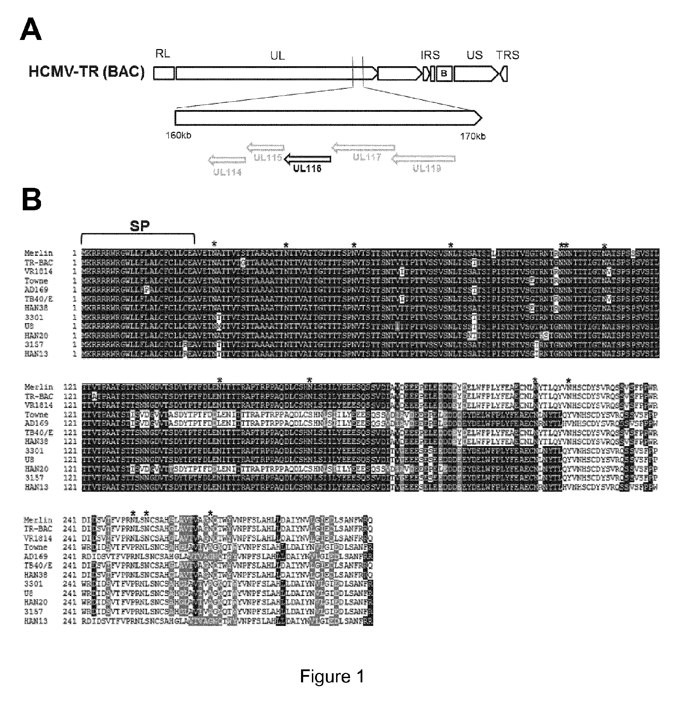

FIG. 1 shows the UL116 position in the HCMV TR genome and sequence conservation among laboratory adapted and clinical HCMV strains. (A) ORF map of the TR BAC clone used in the Examples. Arrows indicate the relative orientations of the repeated and unique ORF blocks. The *ul116* gene, in bold, is located between *ul115* (gL) and *ul117* genes on the antisense strand. (B) T-Coffee primary amino acid sequences multi-alignment showing 98% degree of *ul116* gene conservation among a consistent pool of human CMV strains (from top to bottom, SEQ ID NOS 6, 7, 8, 9, 10, 11, 12, 13, 14, 15, 16, and 17). The asterix indicates the predicted 14 N-glycosilation sites. SP states the N-term predicted signal peptide.

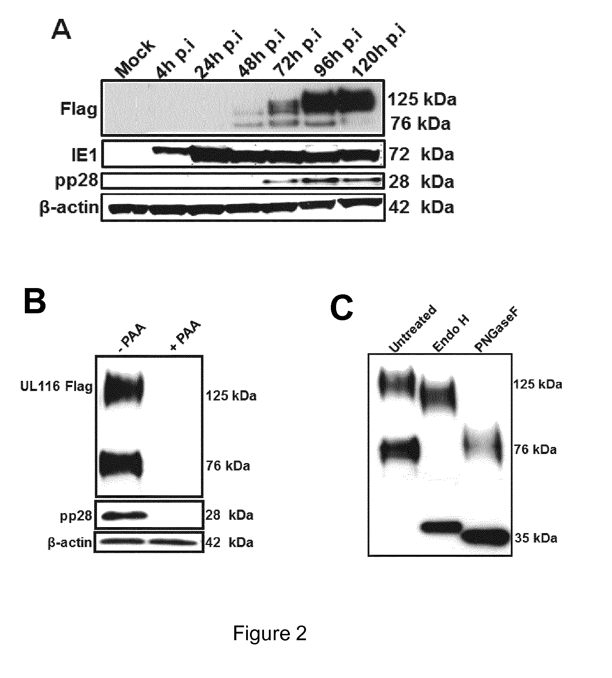

FIG. 2 shows UL116 expression kinetic and charbohydrate addition in human CMV-infected fibroblasts. (A) Uninfected (mock) and TR-UL116-Flag-infected HFF-1cells at an MOI of 5 were harvested at the indicated times p.i. Equivalent amounts of cell lysates were subjected to SDS-PAGE in reducing conditions and analyzed by immunoblotting with anti-Flag, HCMV IE1 and pp28 proteins antibodies as indicated on the left side of the figure. Actin detection was used as a protein loading control. (B) 5 days TR-UL116-Flag infections of HFF were performed in presence (right lane) or absence (left lane) of phosphonacetic acid (PAA), an inhibitor of HCMV late-phase protein expression. Lysates were prepared from infected cells and UL116 expression detected by Western blot using anti-Flag antibody. Detection of pp28 (C) Whole-cell lysate of TR UL116-Flag-infected HFF-1 cells (72 h p.i.) from a single flask was split into three aliquots for glycosidase digestion (left lane, untreated control; middle lane Endoglycosydase H digestion; right lane PNGase F). Proteins were separated on SDS-PAGE in reducing conditions and UL116 revealed by immunoblot analysis using an anti-Flag antibody.

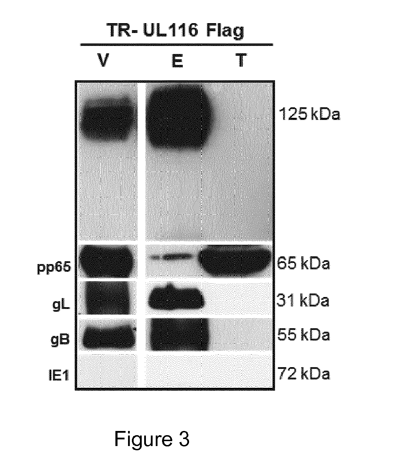

FIG. 3 shows localization of UL116 on the virion envelope. Western blot was performed on purified virions from virus expressing the Flag-tagged UL116. Virions total lysate (V), envelope fraction (E), and tegument/capsid fraction (T) were probed for the antigens indicated.

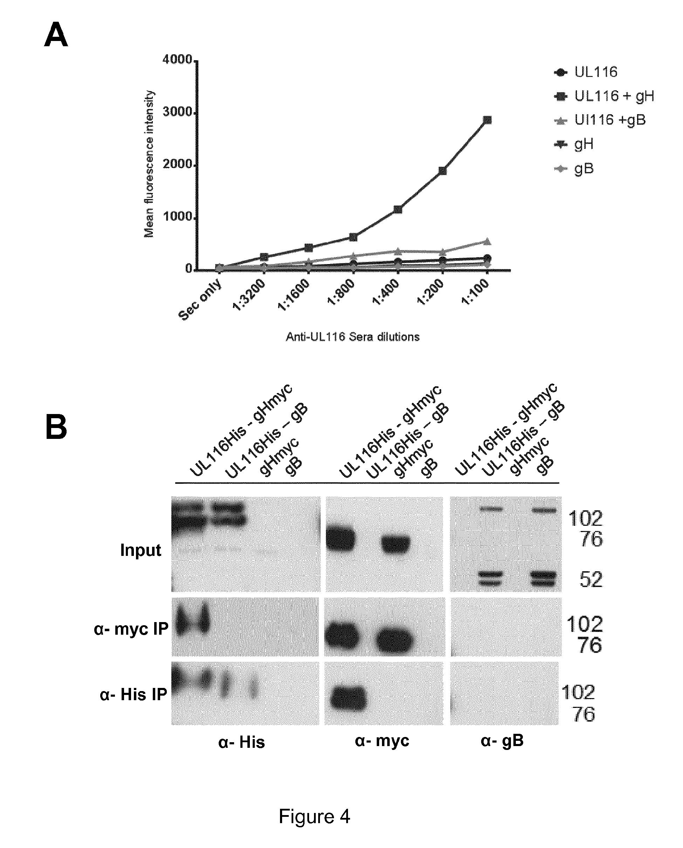

FIG. 4 shows UL116 interaction with gH in HEK 293T transfected cells. (A) Detection of UL116 by FACS analysis on non-permeabilized HEK293T cells is shown. Cells transfected with expression vectors for UL116, gH and gB both alone and in combination as indicated on the figure. 48 hours post-transfection, cells were stained at 4°C with anti-UL116 polyclonal mouse sera at different dilutions. Excess of probe was removed by washing in PBS and then cells were fixed and stained with AlexaFluor-488 conjugated anti-mouse antibody. 488-fluorescent positive cells were found only in the UL116/gH co-transfection setup and compared to the single transfectants for UL116, gH, gB and the couple gB/UL116 used as negative controls. Experiments were performed in triplicate to have a statistically consistent data set. The mean fluorescence intensities plot in figure includes standard deviations. (B) UL116-gH co-immunuprecipitation. Lysates from HEK293T cells transiently expressing UL116-his/gH-myc, UL116-his, gB an UL116-his/gB were subjected to parallel immunoprecipitation experiments (antibodies used specified on the left side of the figure) with both covalently linked magnetic anti-His and anti-myc beads. Total lysates (imput) and eluted samples were separated by SDS-PAGE and analyzed by immunoblot for both the His and myc tag (indicated on the bottom of the figure).

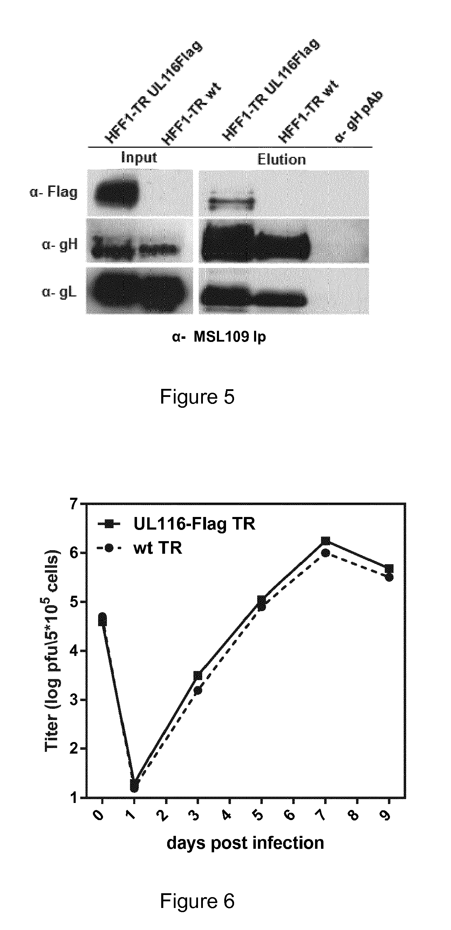

FIG. 5 shows UL116 interaction with gH in infected HFF-1 cells. Co-immunoprecipitation (Co-IP) of gH complexes. Cell lysates were prepared from HFF-1 separately infected with HCMV-TR UL116 Flag and wt TR (5 days p.i.). Complexes were captured using the human anti-gH monoclonal antibody MSL-109 and protein A/G magnetic beads. Total extracts and eluted samples were subjected to immunoblot using an anti-Flag mAb (top panel), a rabbit anti-gH sera (middle panel), and an anti-gL rabbit sera (bottom panel).
FIG. 6 Multiple-step growth curve analysis of the reconstituted virus ORF X-Flag TR and of the parental HCMV strain TR. HFF cells seeded in six-well dishes (5 × 10⁵ cells/well) were infected with an MOI of 0.1. At the indicated time points (days post infection) supernatants from the infected cultures were harvested, and total PFU of infectious virus in the culture supernatants were determined by plaque assay on HFF cells. Time point 0 titers represent input inocula and each data point represents the average of three independent wells.

### DETAILED DESCRIPTION

### 1. gH/UL116 Protein Complex

As disclosed and exemplified herein, the inventors discovered a novel human cytomegalovirus (CMV) protein dimeric complex, formed by CMV proteins gH and UL116.

Accordingly, in one aspect, the invention relates to a recombinant human cytomegalovirus (CMV) protein dimeric complex, comprising CMV gH protein or a complex-forming fragment thereof, and CMV UL116 or a complex-forming fragment thereof. Although gH, UL116, and several other CMV proteins described herein are sometimes referred to as glycoproteins, this nomenclature should not be taken to mean that these proteins must be glycosylated when used with the invention.

### CMV gH proteins

Human CMV glycoprotein H (gH), encoded by the UL75 gene, is a virion glycoprotein that is essential for infectivity and which is conserved among members of the alpha-, beta- and gamma-herpesviruses. Based on the crystal structures of HSV-2 and EBV gH/gL complexes, N-terminal residues of gH form a globular domain at one end of the structure (the "head"), which is implicated in interactions with gB and activation of membrane fusion. The C-terminal domain of gH, proximal to the viral membrane (the "tail"), is also implicated in membrane fusion. gH displays determinants that are recognized by the host factor TLR2, and it directly interacts with a heterodimer formed between the host factors TLR2 and TLR1. TLR2 mediates NF-κB activation and inflammatory cytokine responses from cells.

gH from any CMV strain may be used. By way of examples, gH from CMV strain Merlin is shown as SEQ ID NO: 1 (GI:52139248, 742 amino acid residues). gH from CMV strain Towne is shown as SEQ ID NO: 2 (GI:138314, also 742 amino acid residues), and gH from CMV strain AD169 (GI:138313) is shown as SEQ ID NO: 3. gH from other CMV strain, such as VR1814, Toledo, TR, PH, TB40/e, or Fix (alias VR1814) strain, may also be used.

gH from Towne has been characterized as having: (i) six N-glycosylation sites (at residues 55, 62, 67, 192, 641 and 700); (ii) a hydrophobic signal sequence at its N-terminus (amino acid residues 1-23); (iii) an ectodomain (residues 24-717) that projects out of the cell into the extracellular space; (iv) a hydrophobic transmembrane (TM) domain (residues 718-736); and (v) a C-terminal cytoplasmic domain (residues 737-742). SEQ ID NO: 2 shares 99% and 96% amino acid sequence identity with SEQ ID NO: 1, and SEQ ID NO: 3, respectively.

Typically, the N-terminal signal sequence of full-length gH protein is cleaved by a host cell signal peptidase to produce a mature gH protein. As such, the gH protein expressed by the host cell described herein may lack the N-terminal signal sequence (e.g., gH is encoded by a nucleotide sequence that lacks the coding sequence for the N-terminal signal sequence).

Also encompassed in the invention are complex-forming fragments of gH, such as a gH fragment that lacks the transmembrane (TM) domain (e.g., residues 718-736 of SEQ ID NO:2), the C-terminal domain (e.g., residues 737-742 of SEQ ID NO:2), the N-terminal signal sequence (e.g., residues 1-23 of SEQ ID NO:2), or a combination thereof. A complex-forming fragment of gH can be any part or portion of the gH protein that retain the ability to form a complex with another CMV protein. In certain embodiments, a complex-forming fragment of gH forms part of the dimeric complex gH/UL116. For example, expression of the full-length gH sequence may hinder purification of soluble dimeric complex because the TM domain of gH is hydrophobic. Instead, the gH/UL116 dimeric complex may comprise a gH fragment in which at least a portion of the TM domain of gH deleted.

For example, a gH fragment comprising the N-terminal signal sequence and the ectodomain of gH, but not the TM domain, can be used. A suitable gH fragment may also comprise the a portion of the ectodomain of gH (e.g., at least about 70%, at least about 80%, at least about 85%, at least about 90%, at least about 95%, at least about 96%, at least about 97%, at least about 98%, or at least about 99% of the ectodomain sequence of gH), but none, or only a small portion of the TM domain. Alternatively, the gH fragment described herein may lack between 1 and 20 amino acid residues (e.g. 1, 2, 3, 4, 5, 6, 7, 8, 9, 10, 11, 12, 13, 14, 15, 16, 17, 18, 19 or 20 amino acid residues, or lack 1-20 residues, 1-15 residues, 1-10 residues, 2-20 residues, 2-15 residues, 2-10 residues, 5-20 residues, 5-15 residues, or 5-10 residues) at the N-terminus and/or C-terminus of the full-length ectodomain. Residues at C-terminal domains are believed to be not necessary for immunogenicity. One example of suitable gH fragment described herein is shown as SEQ ID NO: 4, which corresponds to amino acid residues 1-715 of SEQ ID NO: 1. Another example of gH fragment described herein is shown as SEQ ID NO: 5, which lacks the N-terminal signal sequence, TM domain and C-terminal domain of gH, and corresponds to amino acid residues 24-715 of SEQ ID NO: 1. Another example of gH fragment described comprises the entire N-terminal signal sequence and the etcodomain, but lacks the C-terminal domain.

The ectodomain of gH corresponds to the extracellular domain of gH. The location and length of the ectodomain of a gH (or a homologue or a variant thereof) can be predicted based on pairwise alignment of its sequence to SEQ ID NOs: 1, 2, 3, 4, or 5, for example by aligning the amino acid sequence of a gH to SEQ ID NO: 1, and identifying the sequence that aligns to residues 24-717 of SEQ ID NO: 1. Similarly, the locations of the signal sequence, the TM domain, and the C-terminal domain can be predicted by aligning the amino acid sequence of a gH to SEQ ID NOs: 1, 2, 3, 4, or 5, and identifying the sequences that align to the corresponding regions (e.g., residues 1-23 (signal sequence), 718-736 (TM) and 737-742 (C-terminal domain) of SEQ ID NO: 1, respectively). Alternatively, the location and length of the ectodomain, the signal sequence, the TM domain, and the C-terminal domain can be predicted based on computational analysis of the hydrophobicity along the length of a given gH sequence. The signal sequence and the TM domain have the highest levels of hydrophobicity and these two regions flank the ectodomain, which is less hydrophobic.

A suitable complex-forming fragment of gH can also be obtained or determined by standard assays known in the art, such as co-immunoprecipitation assay, cross-linking, or co-localization by fluorescent staining, etc. SDS-PAGE or western blot can also be used (e.g., by showing both subunits are present in a gel electrophoresis). In certain embodiments, the complex-forming fragment of gH (i) forms part of the gH/UL116 dimeric complex; (ii) comprises at least one epitope from SEQ ID NO: 1, SEQ ID NO: 2 , SEQ ID NO: 3, SEQ ID NO: 4, or SEQ ID NO: 5; and/or (iii) can elicit antibodies in vivo which immunologically cross react with a CMV virion.

Other suitable gH proteins can be gH variants that have various degrees of identity to SEQ ID NOs: 1, 2, 3, 4, or 5, such as at least 60%, 70%, 80%, 85%, 90%, 91%, 92%, 93%, 94%, 95%, 96%, 97%, 98% or 99% identical to the sequence recited in SEQ ID NO: 1, SEQ ID NO: 2, SEQ ID NO: 3, SEQ ID NO: 4, or SEQ ID NO: 5. In certain embodiments, the variants of gH (i) forms part of the gH/UL116 dimeric complex; (ii) comprises at least one epitope from SEQ ID NO: 1, SEQ ID NO: 2 , SEQ ID NO: 3, SEQ ID NO: 4, or SEQ ID NO: 5; and/or (iii) can elicit antibodies in vivo which immunologically cross react with a CMV virion.

### CMV UL116 proteins

In all sequenced HCMV genomes, the UL116 ORF is located in the unique-long (UL) region between the UL115 and UL117 genes on the antisense coding strand (Figure 1A). The UL116 mRNA has been shown to arise in the true-late stage of AD169 infection. A multi-alignment of UL116 translation primary sequences derived from gene sequences of HCMV clinical and lab adapted strains is shown in Figure 1B (SEQ ID NOs: 6, 7, 8, 9, 10, 11, 12, 13, 14, 15, 16, and 17). UL116 from all these exemplary CMV strains, as well as from other strains, are suitable for use in the invention. UL116 from Merlin stain (SEQ ID NO: 6), is 313 amino acids long.

Also encompassed in the invention are complex-forming fragments of UL116. A complex-forming fragment of UL116 can be any part or portion of the UL116 protein that retain the ability to form a complex with another CMV protein. In certain embodiments, a complex-forming fragment of UL forms part of the dimeric complex gH/UL116. Suitable fragment of UL116 can be at least 50, at least 60, at least 70, at least 80, at least 90, at least 100, at least 125, at least 150, at least 175, at least 200, at least 225, at least 250, at least 275, or at least 300 amino acids long.

A suitable UL116 fragment may also comprise, e.g., at least about 70%, at least about 80%, at least about 85%, at least about 90%, at least about 95%, at least about 96%, at least about 97%, at least about 98%, or at least about 99% of the full length sequence of UL116. Alternatively, the UL116 fragment described herein may lack between 1 and 20 amino acid residues (e.g. 1, 2, 3, 4, 5, 6, 7, 8, 9, 10, 11, 12, 13, 14, 15, 16, 17, 18, 19 or 20 amino acid residues, or lack 1-20 residues, 1-15 residues, 1-10 residues, 2-20 residues, 2-15 residues, 2-10 residues, 5-20 residues, 5-15 residues, or 5-10 residues) at the N-terminus and/or C-terminus of the full-length UL116.

A suitable complex-forming fragment of UL116 can also be obtained or determined by standard assays known in the art, such as co-immunoprecipitation assay, cross-linking, or co-localization by fluorescent staining, etc. SDS-PAGE or western blot can also be used (e.g., by showing both subunits are present in a gel electrophoresis). In certain embodiments, the complex-forming fragment of UL116 (i) forms part of the gH/UL116 dimeric complex; (ii) comprises at least one epitope from SEQ ID NOs: 6, 7, 8, 9, 10, 11, 12, 13, 14, 15, 16, or 17; and/or (iii) can elicit antibodies in vivo which immunologically cross react with a CMV virion.

Other suitable UL116 proteins can be UL116 variants that have various degrees of identity to SEQ ID NOs: 6, 7, 8, 9, 10, 11, 12, 13, 14, 15, 16, and 17, such as at least 60%, 70%, 80%, 85%, 90%, 91 %, 92%, 93%, 94%, 95%, 96%, 97%, 98% or 99% identical to the sequence recited in SEQ ID NOs: 6, 7, 8, 9, 10, 11, 12, 13, 14, 15, 16, or 17. In certain embodiments, the variants of UL116 (i) forms part of the gH/UL116 dimeric complex; (ii) comprises at least one epitope from comprises at least one epitope from SEQ ID NOs: 6, 7, 8, 9, 10, 11, 12, 13, 14, 15, 16, or 17; and/or (iii) can elicit antibodies in vivo which immunologically cross react with a CMV virion.

### gH/UL 116 Protein Complex

Disclosed herein are dimeric complexes comprising gH (or a complex-forming fragment thereof, or a variant thereof) and UL116 (or a complex-forming fragment thereof, or a variant thereof). For simplicity, the complex is referred to gH/UL116.

A protein complex may also comprise, in addition to gH and UL116 (or complex-forming fragment thereof, or variant thereof), one or more other CMV proteins as part of the complex.

In certain embodiments, to facilitate the assembly of the complex, it may be desirable to express gH and UL116 as a fusion protein. That is, gH (or a complex-forming fragment thereof, or a variant thereof) and UL116 (or a complex-forming fragment thereof, or a variant thereof) are fused into a single polypeptide chain. If one or more additional proteins are also present in the complex, the fusion protein may also comprise such additional protein(s). The subunit can be fused directly, or can be connected by a linker sequence. The linker sequence can be 1 to 50 amino acids long. The linker sequence can be cleavable.

The gH (or a complex-forming fragment thereof, or a variant thereof) and UL116 (or a complex-forming fragment thereof, or a variant thereof) of the invention can include the addition of an amino acid sequence that constitutes a tag, which can facilitate detection (e.g. an epitope tag for detection by monoclonal antibodies) and/or purification (e.g. a polyhistidine-tag to allow purification on a nickel-chelating resin) of the proteins. Examples of affinity-purification tags include, e.g., His tag (hexahistidine (SEQ ID NO: 18), binds to metal ion), maltose-binding protein (MBP) (binds to amylose), glutathione-S-transferase (GST) (binds to glutathione), FLAG tag (Asp-Tyr-Lys-Asp-Asp-Asp-Asp-Lys (SEQ ID NO: 19), binds to an anti-flag antibody), Strep tag (Ala-Trp-Arg-His-Pro-Gln-Phe-Gly-Gly (SEQ ID NO: 20), or Trp-Ser- His-Pro-Gln-Phe-Glu-Lys (SEQ ID NO: 21), bind to streptavidin or a derivative thereof), HA tag, MYC tag, or combination thereof. The tag may be attached to either gH or UL116, or both. One or more tags may be used (e.g., one tag for gH and another for UL116).

### 2. Combination of Antigens

Also provided herein are immunogenic compositions comprising the gH/UL116 complexes described herein. The immunogenic composition may comprise an additional CMV protein or CMV protein complex.

For example, the additional CMV protein or CMV protein complex may be selected from the group consisting of gB, gH, gL, gO, gM, gN; UL128, UL130, UL131, RL10, RL11, RL12, RL13, UL4, UL5, UL10, UL80.5, UL119, UL122, UL133, UL138, UL148A, UL1, UL7, UL9, UL16, UL18, UL20, UL40, UL41A, UL42, UL47, UL111A, UL124, UL132, UL136, UL141, an immunogenic fragment thereof, a complex-forming fragment thereof, and a combination thereof.

In certain embodiments, the additional CMV protein or CMV protein complex may be selected from the group consisting of gB, gH, gL; gO; gM, gN; UL128, UL130, UL131, an immunogenic fragment thereof, a complex-forming fragment thereof, and a combination thereof. For example, the additional CMV protein may be gB or an immunogenic fragment thereof.

In certain embodiments, the additional CMV protein complex is selected from the group consisting of: gH/gL/UL128/UL130/UL131 pentameric complex, gH/gL complex, gH/gL/gO trimeric complex, or a combination thereof. Other suitable complexes include, e.g., gM/gN complex. Each subunit of these complexes can be full length, or a complex-forming fragment thereof. Such complex-forming fragment thereof can be determined by art-known methods as described herein (e.g., by co-immuneoprecipitation, crosslinking, co-localization etc.).

### 3. Recombinant Expression of gH/UL116

Also provided herein are nucleic acids encoding gH (or a complex-forming fragment thereof, or a variant thereof) and UL116 (or a complex-forming fragment thereof, or a variant thereof), as described herein. The nucleic acid may be used directly as a nucleic acid-based vaccine, or can be used for recombinant production of gH/UL116 protein complex. The nucleic acid(s) can be a single construct (e.g., a single vector encoding both gH and UL116), or can be a combination of two or more constructs (e.g., a first vector encoding gH, and a second vector encoding UL116).

The invention also provides a host cell comprising the nucleic acids described herein. When the host cell is cultured under a suitable condition, the nucleic acid(s) can express gH/UL116 protein complex. Preferably, the gH/UL116 protein complex is soluble. Preferably, gH/UL116 protein complex can be secreted from the host cell.

The gH protein itself has a secretory signal. This signal may be used to express the gH/UL116 complex that can be secreted from the host cell. Alternatively or in addition, an appropriate signal peptide may be used in one or more of the five subunits (e.g., by making a fusion protein with a secretory signal). Signal sequences (and expression cassette) for producing secretory proteins are known in the prior art. In general, leader peptides are 5-30 amino acids long, and are typically present at the N-terminus of a newly synthesized protein. The core of the signal peptide generally contains a long stretch of hydrophobic amino acids that has a tendency to form a single alpha-helix. In addition, many signal peptides begin with a short positively charged stretch of amino acids, which may help to enforce proper topology of the polypeptide during translocation. At the end of the signal peptide there is typically a stretch of amino acids that is recognized and cleaved by signal peptidase. Signal peptidase may cleave either during or after completion of translocation to generate a free signal peptide and a mature protein

Suitable host cells include, for example, bacteria (e.g., E. coli, Bacillus subtilis, and Streptococcus spp.), yeast cells (e.g., Saccharomyces cerevisiae, Candida albicans, Candida maltosa, Hansenual polymorpha, Kluyveromyces fragilis, Kluyveromyces lactis, Pichia guillerimondii, Pichia pastoris, Schizosaccharomyces pombe and Yarrowia lipolytica), Tetrahymena cells (e.g., Tetrahymena thermophila), insect cells (e.g., Aedes aegypti, Autographa californica, Bombyx mori, Drosophila melanogaster, Spodoptera frugiperda, and Trichoplusia ni), avian cells (e.g., chicken, duck, and geese), mammalian cells (e.g., human, non-human primate, horse, cow, sheep, dog, cat, and rodent (e.g., hamster)), or combinations thereof.

Suitable insect cell expression systems, such as baculovirus systems, are known to those of skill in the art and described in, e.g., Summers and Smith, Texas Agricultural Experiment Station Bulletin No. 1555 (1987). Materials and methods for baculovirus/insect cell expression systems are commercially available in kit form from, inter alia, Invitrogen, San Diego CA. For example, for expression in insect cells a suitable baculovirus expression vector, such as pFastBac (Invitrogen), is used to produce recombinant baculovirus particles. The baculovirus particles are amplified and used to infect insect cells to express recombinant protein. Suitable insect cells include, for example, Sf9 cells, Sf21 cells, Tn5 cells, Schneider S2 cells, and High Five cells (a clonal isolate derived from the parental Trichoplusia ni BTI-TN-5B1-4 cell line (Invitrogen)).

Avian cell expression systems are also known to those of skill in the art and described in, e.g., U.S. Patent Nos. 5,340,740; 5,656,479; 5,830,510; 6,114,168; and 6,500,668; European Patent No. EP 0787180B; European Patent Application No. EP03291813.8; WO 03/043415; and WO 03/076601. Suitable avian cells include, for example, chicken embryonic stem cells (e.g., EBx® cells), chicken embryonic fibroblasts, chicken embryonic germ cells, duck cells (e.g., AGE1.CR and AGE1.CR.pIX cell lines (ProBioGen) which are described, for example, in Vaccine 27:4975-4982 (2009) and WO2005/042728), EB66 cells, and the like.

Preferably, the host cells are mammalian cells (e.g., human, non-human primate, horse, cow, sheep, dog, cat, and rodent (e.g., hamster). Suitable mammalian cells include, for example, Chinese hamster ovary (CHO) cells, human embryonic kidney cells (HEK-293 cells, typically transformed by sheared adenovirus type 5 DNA), NIH-3T3 cells, 293-T cells, Vero cells, HeLa cells, PERC.6 cells (ECACC deposit number 96022940), Hep G2 cells, MRC-5 (ATCC CCL-171), WI-38 (ATCC CCL-75), fetal rhesus lung cells (ATCC CL-160), Madin-Darby bovine kidney ("MDBK") cells, Madin-Darby canine kidney ("MDCK") cells (e.g., MDCK (NBL2), ATCC CCL34; or MDCK 33016, DSM ACC 2219), baby hamster kidney (BHK) cells, such as BHK21-F, HKCC cells, and the like.

In certain embodiments, the host cell is a CHO cell. In certain embodiments, the polynucleotide encoding the gH (or a complex-forming fragment thereof, or a variant thereof) and UL116 (or a complex-forming fragment thereof, or a variant thereof) described herein is stably integrated into the genomic DNA of the CHO cell.

Various CHO cell lines are also available from European Collection of Cell Cultures (ECACC), or American Type Culture Collection (ATCC), such as CHO cell lines hCBE11 (ATCC® PTA-3357™), E77.4 (ATCC® PTA-3765™), hLT-B: R-hG1 CHO #14 (ATCC® CRL-11965™), MOR-CHO- MORAb-003-RCB (ATCC® PTA-7552™), AQ.C2 clone 11B (ATCC® PTA-3274™), AQ.C2 clone 11B (ATCC® PTA-3274™), hsAQC2 in CHO-DG44 (ATCC® PTA-3356™), xrs5 (ATCC® CRL-2348™), CHO-K1 (ATCC® CCL-61™), Lec1 [originally named Pro-5WgaRI3C] (ATCC® CRL-1735™), Pro-5 (ATCC® CRL-1781™), ACY1-E (ATCC® 65421™), ACY1-E (ATCC® 65420™), pgsE-606 (ATCC® CRL-2246™), CHO-CD36 (ATCC® CRL-2092™), pgsC-605 (ATCC® CRL-2245™), MC2/3 (ATCC® CRL-2143™), CHO-ICAM-1 (ATCC® CRL-2093™), and pgsB-618 (ATCC® CRL-2241 ™). Any one of these CHO cell lines may be used. Exemplary CHO cell lines available at European Collection of Cell Cultures (ECACC) are listed in Table 1.

**Table 1**

| **Cell Line Name** | **Keywords** |
|---|---|
| CHO | Hamster Chinese ovary |
| CHO (PROTEIN FREE) | Chinese hamster ovary |
| CHO-CHRM1 | Human cholinergic receptor muscarinic M1, CHRM1, G Protein Coupled Receptor, GPCR, Transfected, InSCREENeX SCREENflexTM, CHO-K1 Host. |
| CHO-CHRM2 | Human cholinergic receptor muscarinic M2, CHRM2, G Protein Coupled Receptor, GPCR, Transfected, InSCREENeX SCREENflexTM, CHO-K1 Host. |
| CHO-CHRM5 | Human cholinergic receptor muscarinic M5, CHRM5, G Protein Coupled Receptor, GPCR, Transfected, InSCREENeX SCREENflexTM, CHO-K1 Host. |
| CHO-CNR1 | Human cannabinoid receptor I, CNR1 Gene ID 1268, G Protein Coupled Receptor, GPCR, Transfected, InSCREENeX SCREENflexTM, CHO-K1 Host. |
| CHO-FFAR2 | Human free fatty acid receptor 2, FFAR2, G Protein Coupled Receptor, GPCR, Transfected, InSCREENeX SCREENflexTM, CHO-K1 Host. |
| CHO-GPR120 | Human receptor GPR120 (orphan), GPR120, G Protein Coupled Receptor, GPCR, Transfected, InSCREENeX SCREENflexTM, CHO-K1 Host. |
| CHO-K1 | Hamster Chinese ovary |
| CHO-K1-AC-free | Hamster Chinese Ovary, serum-free |
| CHO-K1/SF | Hamster Chinese ovary (MEM adapted) |
| CHO-NPY1 R | Human neuropeptide Y receptor, NPY1R, Gene ID 4886, G Protein Coupled Receptor, GPCR, Transfected, InSCREENeX SCREENflexTM, CHO-K1 Host. |
| CHO-OPRL1 | Human opiate receptor-like 1, OPRL1, G Protein Coupled Receptor, GPCR, Transfected, InSCREENeX SCREENflexTM, CHO-K1 Host. |
| CHO-SSTR1 | Human Somatostatin Receptor 1, SSTR1 G Protein Coupled Receptor, GPCR, Transfected, InSCREENeX SCREENflexTM, CHO-K1 Host. |
| CHO/dhFr- | Hamster Chinese ovary |
| CHO/dhFr-AC-free | Hamster Chinese Ovary, serum-free |
| RR-CHOKI | Hamster Chinese ovary |
| T02J-10/10 (CHO-GCGR (GCGR)) | Human glucagon receptor, GCGR, G Protein Coupled Receptor, GPCR, Transfected, InSCREENeX SCREENflex™, CHO-K1 Host. |

Other commercially available CHO cell lines include, e.g., FreeStyle™ CHO-S Cells and Flp-ln™-CHO Cell Line from Life Technologies.

Methods for expressing recombinant proteins in CHO cells in general have been disclosed. See, e.g., in U.S. Patents No. 4,816,567 and No. 5,981,214.

In certain embodiments, the recombinant nucleic acids are codon optimized for expression in a selected prokaryotic or eukaryotic host cell.

To facilitate replication and expression, the nucleic acids can be incorporated into a vector, such as a prokaryotic or a eukaryotic expression vector. Exemplary vectors include plasmids that are able to replicate autonomously or to be replicated in a host cell. Typical expression vectors contain suitable promoters, enhancers, and terminators that are useful for regulation of the expression of the coding sequence(s) in the expression construct. The vectors may also comprise selection markers to provide a phenotypic trait for selection of transformed host cells (such as conferring resistance to antibiotics such as ampicillin or neomycin).

Exemplary procedures sufficient to guide one of ordinary skill in the art through the production of recombinant nucleic acid(s) for expression of gH/UL116 complex can be found in Sambrook et al., Molecular Cloning: A Laboratory Manual, 2d ed., Cold Spring Harbor Laboratory Press, 1989; Sambrook et al., Molecular Cloning: A Laboratory Manual, 3d ed., Cold Spring Harbor Press, 2001; Ausubel et al., Current Protocols in Molecular Biology, Greene Publishing Associates, 1992 (and Supplements to 2003); and Ausubel et al., Short Protocols in Molecular Biology: A Compendium of Methods from Current Protocols in Molecular Biology, 4th ed., Wiley & Sons, 1999.

Also provided herein is a cell culture comprising the host cell described herein. The cell culture can be large scale, e.g., at least about 10L, least about 20L, least about 30L, least about 40L, at least about 50L, least about 60 L, least about 70L, least about 80L, least about 90L, at least about 100L, least about 150L, least about 200L, at least about 250L, least about 300L, least about 400L, at least about 500L, least about 600L, least about 700L, least about 800L, at least about 900L, at least about 1000 L, at least about 2000 L, at least about 3000 L, at least about 4000 L, at least about 5000 L, at least about 6000 L, at least about 10,000 L, at least about 15,000 L, at least about 20,000 L, at least about 25,000 L, at least about 30,000 L, at least about 35,000 L, at least about 40,000 L, at least about 45,000 L, at least about 50,000 L, at least about 55,000 L, at least about 60,000 L, at least about 65,000 L, at least about 70,000 L, at least about 75,000 L, at least about 80,000 L, at least about 85,000 L, at least about 90,000 L, at least about 95,000 L, at least about 100,000 L, etc.

In certain embodiments, the yield of the gH/UL116 complex from the cell culture is at least about 0.01 g/L, at least about 0.02 g/L, at least about 0.03 g/L, at least about 0.05 g/L, at least about 0.06 g/L, at least about 0.07 g/L, at least about 0.08 g/L, at least about 0.09 g/L, at least about 0.1 g/L, at least about 0.15 g/L, at least about 0.20 g/L, at least about 0.25 g/L, at least about 0.3 g/L, at least about 0.35 g/L, at least about 0.4 g/L, at least about 0.45 g/L, at least about 0.5 g/L, at least about 0.55 g/L, at least about 0.6 g/L, at least about 0.65 g/L, at least about 0.7 g/L, at least about 0.75 g/L, at least about 0.8 g/L, at least about 0.85 g/L, at least about 0.9 g/L, at least about 0.95 g/L, or at least about 1.0 g/L.

Also provided herein is a process of producing a recombinant human cytomegalovirus (CMV) protein dimeric complex, comprising CMV gH protein or a complex-forming fragment thereof, and CMV UL116 or a complex-forming fragment thereof, comprising: (i) culturing the host cell described herein under a suitable condition, thereby expressing said gH/UL116 complex; and (ii) harvesting said gH/UL116 complex from the culture.

In certain embodiments, the gH/UL116 complex described herein is purified. The gH/UL116 complex can be purified using any suitable methods, such as HPLC, various types of chromatography (such as hydrophobic interaction, ion exchange, affinity, chelating, and size exclusion), electrophoresis, density gradient centrifugation, solvent extraction, or the like. As appropriate, gH/UL116 complex may be further purified, as required, so as to remove substantially any proteins which are also secreted in the medium or result from lysis of host cells, so as to provide a product which is at least substantially free of host debris, e.g., proteins, lipids and polysaccharides. See, e.g., those set forth in Sandana (1997) Bioseparation of Proteins, Academic Press, Inc.; and Bollag et al. (1996) Protein Methods, 2nd Edition Wiley-Liss, NY; Walker (1996) The Protein Protocols Handbook Humana Press, NJ, Harris and Angal (1990) Protein Purification Applications: A Practical Approach IRL Press at Oxford, Oxford, U.K.; Scopes (1993) Protein Purification: Principles and Practice 3rd Edition Springer Verlag, NY; Janson and Ryden (1998) Protein Purification: Principles, High Resolution Methods and Applications, Second Edition Wiley-VCH, NY; and Walker (1998) Protein Protocols on CD-ROM Humana Press, NJ. If desired, the gH/UL116 complex can include a "tag" that facilitates purification, as described above.

### 4. PHARMACEUTICAL COMPOSITIONS

The invention provides pharmaceutical compositions and methods of treatment using the cytomegalovirus (CMV) gH/UL116 complex described herein, or a nucleic acid encoding such gH/UL116 complex described herein. For example, the proteins can be delivered directly as components of an immunogenic composition, or nucleic acids that encode the gH/UL116 complex can be administered to produce the CMV protein or immunogenic fragment in vivo. Certain preferred embodiments, such as protein formulations, recombinant nucleic acids (e.g., self-replicating RNA) and alphavirus VRP that contain sequences encoding gH (or a complex-forming fragment thereof, or a variant thereof) and UL116 (or a complex-forming fragment thereof, or a variant thereof) are further described herein.

### Protein Compositions

In one aspect, the invention provides an immunogenic composition comprising the gH/UL116 complex described herein. The composition may comprise one or more additional CMV antigens as described herein.

The immunogenic composition may comprise an adjuvant. Exemplary adjuvants to enhance effectiveness of the composition include: (1) aluminum salts (alum), such as aluminum hydroxide, aluminum phosphate, aluminum sulfate, etc; (2) oil-in-water emulsion formulations (with or without other specific adjuvants such as muramyl peptides (see below) or bacterial cell wall components), such as for example (a) MF59 (PCT Publ. No. WO 90/14837), containing 5% Squalene, 0.5% Tween 80, and 0.5% Span 85 (optionally containing various amounts of MTP-PE (see below), although not required) formulated into submicron particles using a microfluidizer such as Model 110Y microfluidizer (Microfluidics, Newton, Mass.), (b) SAF, containing 10% Squalane, 0.4% Tween 80, 5% pluronic-blocked polymer L121, and thr-MDP (see below) either microfluidized into a submicron emulsion or vortexed to generate a larger particle size emulsion, and (c) RibiTM adjuvant system (RAS), (Ribi Immunochem, Hamilton, Mont.) containing 2% Squalene, 0.2% Tween 80, and one or more bacterial cell wall components from the group consisting of monophosphorylipid A (MPL), trehalose dimycolate (TDM), and cell wall skeleton (CWS), preferably MPL+CWS (DetoxTM); (3) saponin adjuvants, such as StimulonTM (Cambridge Bioscience, Worcester, Mass.) may be used or particles generated therefrom such as ISCOMs (immunostimulating complexes); (4) Complete Freunds Adjuvant (CFA) and Incomplete Freunds Adjuvant (IFA); (5) cytokines, such as interleukins (IL-1, IL-2, etc.), macrophage colony stimulating factor (M-CSF), tumor necrosis factor (TNF), etc; and (6) other substances that act as adjuvants to enhance the effectiveness of the composition.

Muramyl peptides include, but are not limited to, N-acetyl-muramyl-L-threonyl-D-isoglutamine (thr-MDP), N-acetyl-normuramyl-L-alanyl-D-isoglutamine (nor-MDP), N-acety Imuramyl-L-alanyl-D-isoglutaminyl-L-alanine-2-(1'-2'-dipalmitoyl-sn-glycer o-3-hydroxyphosphoryloxy)-ethylamine (MTP-PE), etc.

In certain embodiment, the adjuvant is an oil-in-water emulsion, such as MF59. In certain embodiment, the adjuvant is a TLR7 agonist, such as imidazoquinoline or imiquimod. In certain embodiment, the adjuvant is an aluminum salt, such as aluminum hydroxide, aluminum phosphate, aluminum sulfate.

The adjuvants described herein can be used singularly or in any combination, such as alum/TLR7 agonist combination.

### Nucleic Acid Vaccines and Delivery Systems

Recombinant nucleic acid molecule that encodes the CMV gH/UL116 complex described herein can be administered to induce production of the encoded gH and UL116 proteins, and an immune response thereto.

The recombinant nucleic acid can be DNA (e.g., plasmid or viral DNA) or RNA, preferably self-replicating RNA, and can be monocystronic or polycistronic. Any suitable DNA or RNA can be used as the nucleic acid vector that carries the open reading frames that encode the gH/UL116 complex described herein. Suitable nucleic acid vectors have the capacity to carry and drive expression of individual subunit of the gH/UL116 complex (as well as any other CMV antigens, if present). Such nucleic acid vectors are known in the art and include, for example, plasmids, DNA obtained from DNA viruses such as vaccinia virus vectors (e.g., NYVAC, see US 5,494,807), and poxvirus vectors (e.g., ALVAC canarypox vector, Sanofi Pasteur), and RNA obtained from suitable RNA viruses such as alphavirus. If desired, the recombinant nucleic acid molecule can be modified, e.g., contain modified nucleobases and or linkages as described further herein.

The self-replicating RNA molecules of the invention are based on the genomic RNA of RNA viruses, but lack the genes encoding one or more structural proteins. The self-replicating RNA molecules are capable of being translated to produce non-structural proteins of the RNA virus and CMV gH and UL116 proteins (or fragments or variants0 encoded by the self-replicating RNA.

The self-replicating RNA generally contains at least one or more genes selected from the group consisting of viral replicase, viral proteases, viral helicases and other nonstructural viral proteins, and also comprise 5'- and 3'-end cis-active replication sequences, and a heterologous sequences that encodes one or more CMV antigens (e.g., gH (or fragment or variants thereof), UL116 (or fragment or variants thereof), and/or any additional desired CMV protein antigens). A subgenomic promoter that directs expression of the heterologous sequence(s) can be included in the self-replicating RNA. If desired, a heterologous sequence may be fused in frame to other coding regions in the self-replicating RNA and/or may be under the control of an internal ribosome entry site (IRES) and/or 2A sequence, or a combination thereof. Nucleotide sequences encoding gH (or fragment or variants thereof), UL116 (or fragment or variants thereof), and any additional desired CMV protein antigens if present, can be delivered by a single RNA vector (e.g., bicistronic RNA or polycistronic RNA), or can be delivered by separate RNA vectors. If delivered as bicistronic RNA or polycistronic RNA, IRES and/or 2A sequences may be used produce individual proteins.

Self-replicating RNA molecules of the invention can be designed so that the self-replicating RNA molecule cannot induce production of infectious viral particles. This can be achieved, for example, by omitting one or more viral genes encoding structural proteins that are necessary for the production of viral particles in the self-replicating RNA. For example, when the self-replicating RNA molecule is based on an alpha virus, such as Sinbis virus (SIN), Semliki forest virus and Venezuelan equine encephalitis virus (VEE), one or more genes encoding viral structural proteins, such as capsid and/or envelope glycoproteins, can be omitted. As used herein, the term "alphavirus" has its conventional meaning in the art and includes various species such as Venezuelan equine encephalitis virus (VEE; e.g., Trinidad donkey, TC83CR, etc.), Semliki Forest virus (SFV), Sindbis virus, Ross River virus, Western equine encephalitis virus, Eastern equine encephalitis virus, Chikungunya virus, S.A. AR86 virus, Everglades virus, Mucambo virus, Barmah Forest virus, Middelburg virus, Pixuna virus, O'nyong-nyong virus, Getah virus, Sagiyama virus, Bebaru virus, Mayaro virus, Una virus, Aura virus, Whataroa virus, Banbanki virus, Kyzylagach virus, Highlands J virus, Fort Morgan virus, Ndumu virus, and Buggy Creek virus.

A self-replicating RNA molecule can, when delivered to a vertebrate cell even without any proteins, lead to the production of multiple daughter RNAs by transcription from itself (or from an antisense copy of itself). The self-replicating RNA can be directly translated after delivery to a cell, and this translation provides a RNA-dependent RNA polymerase which then produces transcripts from the delivered RNA. Thus the delivered RNA leads to the production of multiple daughter RNAs. These transcripts are antisense relative to the delivered RNA and may be translated themselves to provide in situ expression of encoded CMV protein, or may be transcribed to provide further transcripts with the same sense as the delivered RNA which are translated to provide in situ expression of the encoded CMV protein(s).

A preferred self-replicating RNA molecule thus encodes (i) a RNA-dependent RNA polymerase which can transcribe RNA from the self-replicating RNA molecule and (ii) gH (or fragment or variants thereof), UL116 (or fragment or variants thereof), and any additional desired CMV protein antigens if present. The polymerase can be an alphavirus replicase e.g. comprising alphavirus non-structural proteins nsP1-nsP4.

The self-replicating RNA molecules of the invention can contain one or more modified nucleotides and therefore have improved stability and be resistant to degradation and clearance in vivo, and other advantages. There are more than 96 naturally occurring nucleoside modifications found on mammalian RNA. See, e.g., Limbach et al., Nucleic Acids Research, 22(12):2183-2196 (1994). The preparation of nucleotides and modified nucleotides and nucleosides are well-known in the art, e.g. from US Patent Numbers 4373071, 4458066, 4500707, 4668777, 4973679, 5047524, 5132418, 5153319, 5262530, 5700642 all of which are incorporated herein by reference in their entirety, and many modified nucleosides and modified nucleotides are commercially available. If desired, the self-replicating RNA molecule can contain phosphoramidate, phosphorothioate, and/or methylphosphonate linkages.

The self-replicating RNA described herein is suitable for delivery in a variety of modalities, such as naked RNA delivery or in combination with lipids, polymers or other compounds that facilitate entry into the cells. Self-replicating RNA molecules can be introduced into target cells or subjects using any suitable technique, e.g., by direct injection, microinjection, electroporation, lipofection, biolystics, and the like. The self-replicating RNA molecule may also be introduced into cells by way of receptor-mediated endocytosis. See e.g., U.S. Pat. No. 6,090,619; Wu and Wu, J. Biol. Chem., 263:14621 (1988); and Curiel et al., Proc. Natl. Acad. Sci. USA, 88:8850 (1991). For example, U.S. Pat. No. 6,083,741 discloses introducing an exogenous nucleic acid into mammalian cells by associating the nucleic acid to a polycation moiety (e.g., poly-L-lysine having 3-100 lysine amino acids), which is itself coupled to an integrin receptor-binding moiety (e.g., a cyclic peptide having the sequence Arg-Gly-Asp).

The self-replicating RNA molecules can be delivered into cells via amphiphiles. See e.g., U.S. Pat. No. 6,071,890. Typically, a nucleic acid molecule may form a complex with the cationic amphiphile. Mammalian cells contacted with the complex can readily take it up.

The self-replicating RNA can be delivered as naked RNA (e.g. merely as an aqueous solution of RNA) but, to enhance entry into cells and also subsequent intercellular effects, the self-replicating RNA is preferably administered in combination with a delivery system, such as a particulate or emulsion delivery system. A large number of delivery systems are well known to those of skill in the art. Three particularly useful delivery systems are (i) liposomes, (ii) non-toxic and biodegradable polymer microparticles, and (iii) cationic submicron oil-in-water emulsions. Non-virion based delivery, wherein the RNA is not packaged into a virion particle, generally has better safety profiles.

If desired, self-replicating RNA molecules of the invention can be designed to induce production of infectious viral particles that are attenuated or virulent, or to produce viral particles that are capable of a single round of subsequent infection.

The invention also provides immunogenic composition comprising the nucleic acid (e.g., self-replicating RNA) described herein. The immunogenic composition may comprise an adjuvant, as described above. Preferred adjuvants include, e.g., an aluminum salt or an oil-in-water emulsion (such as MF59).

### Alphavirus VRPs

In some embodiments, the CMV gH/UL116 complexes described herein are delivered using alphavirus replicon particles (VRP).

An "alphavirus replicon particle" (VRP) or "replicon particle" is an alphavirus replicon packaged with alphavirus structural proteins. An "alphavirus replicon" (or "replicon") is an RNA molecule which can direct its own amplification in vivo in a target cell. The replicon encodes the polymerase(s) which catalyze RNA amplification (nsPI, nsP2, nsP3, nsP4) and contains cis RNA sequences required for replication which are recognized and utilized by the encoded polymerase(s). An alphavirus replicon typically contains the following ordered elements: 5' viral sequences required in cis for replication, sequences which encode biologically active alphavirus nonstructural proteins (nsP1, nsP2, nsP3, nsP4), 3' viral sequences required in cis for replication, and a polyadenylate tract. An alphavirus replicon also may contain one or more viral subgenomic "junction region" promoters directing the expression of heterologous nucleotide sequences, which may, in certain embodiments, be modified in order to increase or reduce viral transcription of the subgenomic fragment and heterologous sequence(s) to be expressed. Other control elements can be used, such as IRES or 2A sequences.

### Pharmaceutical Formulations

Each of the immunogenic compositions discussed herein may be used alone or in combination with one or more other antigens, the latter either from the same viral pathogen or from another pathogenic source or sources. These pharmaceutical formulations may either be prophylactic (to prevent infection) or therapeutic (to treat disease after infection).

Such pharmaceutical formulations comprise an immunogenic composition, usually in combination with "pharmaceutically acceptable carriers," which include any carrier that does not itself induce the production of antibodies harmful to the individual receiving the composition. Suitable carriers are typically large, slowly metabolized macromolecules such as proteins, polysaccharides, polylactic acids, polyglycolic acids, polymeric amino acids, amino acid copolymers, lipid aggregates (such as oil droplets or liposomes), and inactive virus particles. Such carriers are well known to those of ordinary skill in the art. Additionally, these carriers may function as adjuvants. Furthermore, the antigen may be conjugated to a bacterial toxoid, such as a toxoid from diphtheria, tetanus, cholera, H. pylori, etc. pathogens.

The pharmaceutical formulations may comprise an adjuvant, as described above.

The pharmaceutical formulations (e.g., the immunogenic composition, pharmaceutically acceptable carrier, and adjuvant) typically will contain diluents, such as water, saline, glycerol, ethanol, etc. Additionally, auxiliary substances, such as wetting or emulsifying agents, Ph buffering substances, and the like, may be present in such vehicles.

Typically, the pharmaceutical formulations are prepared as injectables, either as liquid solutions or suspensions; solid forms suitable for solution in, or suspension in, liquid vehicles prior to injection may also be prepared. The preparation also may be emulsified or encapsulated in liposomes for enhanced adjuvant effect, as discussed above under pharmaceutically acceptable carriers.

Pharmaceutical formulations comprise an immunologically effective amount of the antigenic polypeptides, as well as any other of the above-mentioned components, as needed. By "immunologically effective amount," it is meant that the administration of that amount to an individual, either in a single dose or as part of a series, is effective for treatment or prevention of illness, infection or disease. This amount varies depending upon the health and physical condition of the individual to be treated, the taxonomic group of individual to be treated (e.g., nonhuman primate, primate, etc.), the capacity of the individual's immune system to synthesize antibodies, the degree of protection desired, the formulation of the vaccine, the treating doctor's assessment of the medical situation, and other relevant factors. It is expected that the amount will fall in a relatively broad range that can be determined through routine trials.

The pharmaceutical formulations are conventionally administered parenterally, e.g., by injection, either subcutaneously or intramuscularly. Additional formulations suitable for other modes of administration include oral and pulmonary formulations, suppositories, and transdermal applications. Oral formulations may be preferred for certain viral proteins. Dosage treatment may be a single dose schedule or a multiple dose schedule. The immunogenic composition may be administered in conjunction with other immunoregulatory agents.

### 5. Methods of Treatment

In another aspect, the invention provides a method of inducing an immune response against cytomegalovirus (CMV), comprising administering to a subject in need thereof an immunologically effective amount of the immunogenic composition describe herein, which comprises the proteins, DNA molecules, RNA molecules (e.g., self-replicating RNA molecules), or VRPs as described above.

In certain embodiments, the immune response comprises the production of neutralizing antibodies against CMV. In certain embodiments, the neutralizing antibodies are complement-independent.

The immune response can comprise a humoral immune response, a cell-mediated immune response, or both. In some embodiments an immune response is induced against each delivered CMV protein. A cell-mediated immune response can comprise a Helper T-cell (Th) response, a CD8+ cytotoxic T-cell (CTL) response, or both. In some embodiments the immune response comprises a humoral immune response, and the antibodies are neutralizing antibodies. Neutralizing antibodies block viral infection of cells. CMV infects epithelial cells and also fibroblast cells. In some embodiments the immune response reduces or prevents infection of both cell types. Neutralizing antibody responses can be complement-dependent or complement-independent. In some embodiments the neutralizing antibody response is complement-independent. In some embodiments the neutralizing antibody response is cross-neutralizing; i.e., an antibody generated against an administered composition neutralizes a CMV virus of a strain other than the strain used in the composition.

A useful measure of antibody potency in the art is "50% neutralization titer." To determine 50% neutralizing titer, serum from immunized animals is diluted to assess how dilute serum can be yet retain the ability to block entry of 50% of viruses into cells. For example, a titer of 700 means that serum retained the ability to neutralize 50% of virus after being diluted 700-fold. Thus, higher titers indicate more potent neutralizing antibody responses. In some embodiments, this titer is in a range having a lower limit of about 200, about 400, about 600, about 800, about 1000, about 1500, about 2000, about 2500, about 3000, about 3500, about 4000, about 4500, about 5000, about 5500, about 6000, about 6500, or about 7000. The 50% neutralization titer range can have an upper limit of about 400, about 600, about 800, about 1000, about 1500, about 2000, about 2500, about 3000, about 3500, about 4000, about 4500, about 5000, about 5500, about 6000, about 6500, about 7000, about 8000, about 9000, about 10000, about 11000, about 12000, about 13000, about 14000, about 15000, about 16000, about 17000, about 18000, about 19000, about 20000, about 21000, about 22000, about 23000, about 24000, about 25000, about 26000, about 27000, about 28000, about 29000, or about 30000. For example, the 50% neutralization titer can be about 3000 to about 25000. "About" means plus or minus 10% of the recited value. Neutralization titer can be measured as described in the specific examples, below.

An immune response can be stimulated by administering proteins, DNA molecules, RNA molecules (e.g., self-replicating RNA molecules), or VRPs to an individual, typically a mammal, including a human. In some embodiments the immune response induced is a protective immune response, i.e., the response reduces the risk or severity of CMV infection. Stimulating a protective immune response is particularly desirable in some populations particularly at risk from CMV infection and disease. For example, at-risk populations include solid organ transplant (SOT) patients, bone marrow transplant patients, and hematopoietic stem cell transplant (HSCT) patients. VRPs can be administered to a transplant donor pre-transplant, or a transplant recipient pre- and/or post-transplant. Because vertical transmission from mother to child is a common source of infecting infants, administering VRPs to a woman who is pregnant or can become pregnant is particularly useful.

Any suitable route of administration can be used. For example, a composition can be administered intramuscularly, intraperitoneally, subcutaneously, or transdermally. Some embodiments will be administered through an intra-mucosal route such as intra-orally, intra-nasally, intra-vaginally, and intra-rectally. Compositions can be administered according to any suitable schedule.

Also provided herein is a method of inhibiting cytomegalovirus (CMV) entry into a cell, comprising contacting the cell with the immunogenic composition described herein.

This invention is further illustrated by the following examples which should not be construed as limiting.

### EXAMPLES

### Example 1. MATERIALS & METHODS

### Cell lines, plasmids and viruses.

TR is a clinical human CMV strain that was derived from a ocular vitreous fluid sample from a patient with HIV disease and was cloned into a BAC after limited passage in fibroblasts.

HCMV strain TR and recombinant UL116 Flag-TR virus were propagated in human foreskin fibroblasts HFF-1 (ATCC: SCRC-1041) grown in minimal essential medium (Invitrogen) supplemented with 10% fetal calf serum, glutamine (100 mg/liter), and gentamicin (350 mg/liter). Virions were isolated by glycerol-tartrate gradient centrifugation as described. HEK293T cells were cultured in Dulbecco's modified Eagle's medium supplemented with 10% fetal calf serum, glutamine and gentamicin. Lipofectamine 2000 (Invitrogen) was used to transfect HEK293T cells. Human codon-optimized UL116, gH(UL75), gL(UL115) and gB (UL55) HCMV genes based on the TR strain sequence were synthesized by Geneart and cloned in plasmid pcDNA3.1(-)/myc-His C (Invitrogen) in frame with C-terminal myc and six histidine tag sequences. Single point-mutations were performed with the Quick Change Mutagenesis Kit (Stratagene) resulting in the mono-tag versions and the tag-less versions of these genes.

### Construction and generation of UL116-Flag TR virus.

Insertion of the Flag tag at the C-terminus of UL116 ORF on the TR BAC was achieved using the method of Two-step Red-mediated recombination. The primer pair used to amplify the kanamycin insertion cassette are the forward 5'-TTC GGC GCC AAC TGG CTC CTT ACC GTC ACA CTC TCA TCG TGC CGC AGA CTG ATT ACA AGG ATG ACG ACG ATA AG-3' (SEQ ID NO: 22) and the reverse 5'-TAT CAC CGG TCC AGG TGA GAA AGA GAA GCC GCA ATC CGG GCG GCG GCA CA TCA CTT ATC GTC GTC ATC CTT GTA ATC AGT CTG CGG CAC GAT GAG CAA CCA AAT TAA ACCA ATT CTG ATT TAG-3' (SEQ ID NO: 23) where the underlined base pairs encode for the Flag peptide.

### Reconstitution of infectious virus.

To reconstitute the virus, 2 ug of the BAC-HCMV DNA and 1 ug of the pp71 expression plasmid were transfected into MRC-5 cells by electroporation as described in the current protocols. Culture medium was changed 24 h later and the cells were split and cultured until the appearance of virus plaques.

The virus stock was prepared by harvesting cell-free culture supernatant when the entire monolayer of cells was lysed or the cells were split and cultured until the appearance of virus plaques.

### Purification of HCMV-TR virions.

Human CMV particles in cell supernatants were separated into virion, dense body, and noninfectious enveloped particle (NIEP) fractions by positive density/negative viscosity gradient centrifugation as described previously. Particle concentrations in the preparations were estimated by counting negatively stained samples by EM in relation to a standard concentration of latex beads. To separate virion envelope proteins from capsid and tegument proteins, 10⁸ particles were mixed 1:1 with envelope stripping buffer (2% Nonidet-P40 in PBS) and incubated for 15 minutes at 4°C. Particles were pelleted (12,000 g for 5 minutes at 4°C), and the soluble envelope fraction was harvested. The insoluble capsid/tegument material was washed twice with envelope-stripping buffer and once in PBS before being solubilized in SDS-PAGE sample buffer.

### Flow cytometry.

For detection of membrane exposed UL116, HEK293T transiently transfected with vectors coding for UL116, gH, gB and empty vector were trypsin detached 48 h post-transfection, incubated 30 min at RT with Live&Dead Agua (Invitrogen), diluted 1:400 in PBS then incubated with different dilutions of anti-UL116 polyclonal mouse sera for 60 min on ice. As a secondary antibody, Alexa Fluor 647-conjugated goat anti-mouse was used for 30 min on ice at 1:200 dilution. A total of 10⁴ cells were analyzed for each curve using FACSCanto II (Becton Dickinson, Heidelberg, Germany). Experiments were performed in triplicate for statistical consistency, means and standard deviations were analyzed and plotted using Graphpad Prism software.

### Glycosidase treatment.

Samples were treated with PNGase F (P0705S; New England BioLabs) or ENDO H (P0703S; New England BioLabs) according to the manufacturer's instructions. Briefly, the samples were denatured in glycoprotein-denaturing buffer at 100°C for 10 minutes and cooled to 0°C for 5 minutes. The samples were then digested overnight at 37°C with PNGase F or ENDO H before being analyzed by Western immunoblotting.

### Immunogold electron microscopy.

Purified virions were air-dried to the surface of Formvar-coated EM grids. The grids were treated with mouse Anti-Flag antibody (Sigma-F3165) for 4 hours at room temperature, washed 3 times with PBS, and incubated with goat anti-mouse antibody conjugated to 5-nm gold particles for 1 hour at room temperature. After further washing in PBS, the grids were negatively stained with phosphotungstic acid and subjected to EM.

### Purification of gH/UL116 and gH/gLC144A-3G16 complexes:

The gH/UL116 complex was purified from supernatants of HEK-293EBNA cells double transfected with plasmids encoding UL116 tagged at the C-terminus with a double strep-tag and untagged gH. Following Strep-tag affinity chromatography purification, the complex was incubated with the 3G16-Fab. The trimeric complex gH/UL116/3G16-Fab was further purified by Size Exclusion Chromatography (SEC). The gH/gL complex harboring the mutation gL-C144A mutation was purified from supernatants of HEK-293EBNA cells double transfected with plasmids encoding a C-terminally His tagged gH and untagged gL as previously described (Ciferri et al., submitted). Purified gH/gL-C144A was mixed 1:1.2 ratio with 3G16 and the ternary complex isolated by SEC.

### Negative Staining Electron Microscopy and single particle analysis:

Five microliters of purified gH/UL116-3G16 and gH/gL(C144A)-3G16 samples (approximately 100 ng) were placed onto a freshly glow discharged holey carbon grid covered with a thin layer of continuous carbon. The grid was stained with sequential 75-µl drops of a freshly prepared 2% (w/v) uranyl formate solution. Samples were imaged using a Tecnai Spirit T12 transmission electron microscope operating at 120 keV at a nominal magnification of 49,000× (1.57 Å/pixel at the detector level) using a defocus range of -0.8 to -1.2 µm. Images were recorded under low-dose conditions on a Gatan 4096 × 4096 pixel CCD camera.

Particles were manually picked using e2boxer (EMAN2,) and extracted using a 224 pixels box size. The two datasets were band-pass filtered with a 200-Å high-pass cutoff and a 20-Å low-pass cutoff. For 2D classification, we used iterative multivariate statistical analysis (MSA) and multi-reference alignment (MRA) in Imagic. 2D classes from gH/gL and gH/Ul116 were aligned and compared by cross-correlation using the SPIDER 'AP SH' command.

### SDS-PAGE and immunoblotting.

Proteins were separated by sodium dodecyl sulfate-polyacrylamide gel electrophoresis (SDS-PAGE) on 10 to 15% polyacrylamide gels under standard conditions. Proteins were transferred to nitrocellulose membranes, and membranes were blocked with PBS containing 0.1 % Tween 20 and 5% powdered milk. Antibodies and sera were diluted in PBS containing 0.1 % Tween 20. For detection of primary antibody binding, horseradish peroxidase- conjugated anti-rabbit or anti-mouse immunoglobulin G antibody (Perkin Elmer) and the enhanced chemiluminescence detection system (Pierce) were used according to the manufacturer's instructions. Late-phase proteins expression was inhibited by the use of phosphonoacetic acid (PAA; Sigma-Aldrich). A total of 250 µg/ml of PAA was added to the medium at the time of infection and maintained throughout infection.

### Immunofluorescence.

Cells were plated on glass coverslips and infected with HCMV. At 7 days PI, the cells were fixed in 4% paraformaldehyde, permeabilized with 0.5% NP-40, pre-blocked with HCMV seronegative human IgG and incubated with primary antibody for 1 hour at RT°C. Following washing, secondary antibodies were incubated for 1 hour at 37°C, washed again, and mounted with DAPI Pro-long Safe Stain mounting media (Invitrogen). Primary antibodies were rabbit anti-Flag (F7425; Sigma), mouse anti Flag (F1804-Sigma), sheep anti-human TGN46 (AHP500; Serotec), mouse MAb anti-pp28 (6502; Abcam) mouse anti-gH (2470-5437; Adb Serotech) and rabbit anti gL (1260A-OHSU). Secondary antibodies were Alexa Fluor 488-, 568-, and 647-conjugated goat anti-mouse and anti-rabbit (Invitrogen). The intracellular locations of antibody-tagged proteins were examined under laser illumination in a Zeiss LMS 700 confocal microscope, and images were captured using ZEN 2009 software.

### Immunoprecipitations.

HFF-1 cells were infected with wtTR and UL-116Flag-TR stocks. Protein expression was allowed to proceed for 72hrs before the cells were washed in 1xPBS (0.137M NaCl, 0.0027M KCI, 0.1 M, Na2HPO4, 0.002M KH2PO4, pH7.4) and lysed (0.025M Tris, 0.15M NaCl, 0.001M EDTA, 1%NP-40, 5% glycerol, pH 7.4) in the presence of protease inhibitors. 500 µg of protein was incubated overnight at 4°C with 5µg of MSL-109 (courtesy of A.Feire) monoclonal antibody. Complexes were immunoprecipitated using the Protein G Dynalbeads (Invitrogen) according to the manufacturer's protocol. The complexes were washed four times in lysis buffer. Samples were boiled for 5 mins before immunoblotting. The same procedure described above was adopted for the immunoprecipitation experiments carried out on transiently expressing HEK293T cells. Complexes were captured in parallel experiments with covalently linked anti-His ab magnetic beads (Genescript) and anti-c-myc magnetic beads (Pierce) to avoid background signals in resulting eluted materials.

### Example 2. RESULTS

### Primary structure of UL116 gene product

In all sequenced human CMV genomes, the *ul116* gene is located in the unique-long (UL) region between the *ul115* (gL) and *ul117* genes on the antisense coding strand (FIG. 1A). The *ul116* mRNA was previously shown to arise in the true-late stage of AD169 infection as part of the UL119-UL115 transcription unit but the gene product never analyzed. A multi-alignment of *ul116* translation primary sequences derived from gene sequences of HCMV clinical and lab adapted strains is shown in FIG. 1B. The conservation degree among most representative human CMV strains is 98% identity resulting in a very strong conservation of this protein among the cytomegalovirus population. The *ul116* ORF is predicted to encode a 313 amino-acid glycoprotein (UL116) comprising a signal-peptide (amino acid position 1-24, SP in FIG. 1 B), a threonine-rich domain (amino acid 27-157) and 14 predicted N-linked glycosylation consensus sites. The resulting polypeptide backbone is predicted to have a molecular mass of 34.2 kDa (added of 3 kDa on the viral FLAG-tagged protein and of about 6 kDa for myc-HisUL116). Moreover, UL116 lacks membrane anchor sequences and is expected to be a secreted protein.

### Kinetics of UL116 expression during HCMV replication.

To investigate the expression kinetics of UL116 during productive human CMV infection we generated a recombinant human CMV (TR-derived BAC) with a Flag-tag fused to the UL116 C-terminal end (see materials and methods). The reconstituted TR UL116-Flag virus was used to infect HFF-1 cells and the expression kinetic of UL116 monitored by immunoblot using an anti-Flag antibody. Infected cells were harvested at different time points, ranging from 4 to 120 h p.i., and extracts prepared. As control, expression of the major immediate protein IE1 pp72 (UL123) and of the late pp28 (UL99) phosphoprotein were revealed in the same samples. Results are shown in FIG. 2A. At time points after infection, when signals for UL116 were not observed, the immediate early protein IE1/pp72 was present. As described in the literature, IE1 begun to be revealed at 4 h p.i. and it was present throughout the entire HCMV replication cycle. Detection of UL116 expression went in parallel with pp28, a late phase marker. Consistently with the observed kinetic pattern, a metabolic blockade with PAA resulted in the disappearance of the UL116 bands (FIG. 2B). In extracts of infected human fibroblasts, UL116 migrated as two species, a faster-migrating protein of 76 kDa and a slower migrating protein of about 125 kDa (FIG. 2A). The latter appeared to be the mature product of the 76 kDa species as indicated by the increased accumulation of the 125 kDa species from 72 h p.i. through 120 h p.i. not observed for the faster migrating band (FIG. 2A).

Trafficking of herpesvirus glycoproteins to the Golgi apparatus is associated with the processing of N-linked oligosaccharides to complex oligosaccharides. Extensive modifications of the sugar content generate carbohydrate chains that are resistant to the action of the endoglycosydase H (endoH). EndoH completely cleaves only ER-type N-linked carbohydrates while hybrid and complex high mannose N-linked carbohydrates, products of golgi sugar trimming and additions, are cleaved by the action of Peptide -*N*-Glycosidase F (PNGase F). The high number of putative N-linked glycan sites (n = 14) predicted on UL116, the difference between the apparent molecular mass observed (125 kDa) by immunoblotting and the calculated molecular mass (34.2 kDa) of the 313-aminoacid polypeptide backbone suggested that UL116 undergoes an extensive post-translational glycosylation process. To verify the glycosylation status of UL116 during viral life cycle, HFF were infected with TR UL116-Flag for 3 days before to harvest cells and perform glycosidases digestion on the extract. As shown in FIG. 2C, digestion with endoH have different outcomes on the two species of UL116: a) the 125 kDa band is poorly affected and it reduced its apparent MW of about 10 kDa; b) the 75 kDa band collapsed to -38 kDa, a value very close to the predicted MW based on sequence (FIG. 2C compare lane 1 and 2). PNGase F digestion caused the disappearance of the faster migrating 76 kDa band that migrated to approximately 35 kDa while the apparent MW of the 125 kDa band was reduced at -78 kDa (Fig 2C, compare lanes 1 and 3). This result is consistent with the presence of a post-golgi carbohydrates modification.

### Confocal analysis of pUL116 in infected human fibroblasts.

Confocal microscopy was used in order to investigate the localization of UL116 in HCMV-infected cells. Anti-Flag antibody was employed to trace subcellular distribution of UL116 together with antibodies specific for cellular compartments or different HCMV proteins. Human fibroblasts were infected with TR UL116-Flag and at 96 h p.i. fixed and stained for confocal analysis.

Among the cellular markers used, UL116 showed a strong co-localization with the Trans-Golgi marker TGN 46. Based on the cytoplasmic compartmentalization of UL116, we explored whether other structural HCMV proteins gathered at the UL116-containing site. To this purpose, in HFF-1 TR UL116-Flag infected cells following fixation at 72 h p.i., antibodies against the tegument phosphoprotein pp28 and the envelope glycoprotein gL were employed. Both HCMV proteins were previously reported to localize with other tegument (pp28) or envelope (gL) proteins at the virus assembly complex (AC) site during the late phase of the infectious cycle and to be acquired by the sorting virion. Based on confocal microscopy, we found a robust co-localization of pUL116 with pp28 and gL. These data are consistent with the trafficking of UL116 to the site of viral AC and led us to hypothesize that it could be inserted on the viral particle.

### UL116 is a component of the HCMV virion envelope.

To verify if UL116 was incorporated into the virion and to establish its localization we purified the recombinant TR UL116-Flag viral particles from the supernatant of infected HFF and performed both Western blot and electron microscopy analysis.

TR UL116-Flag particles purified by negative-viscosity-positive-glycerol/tartrate gradient centrifugation were submitted to detergent extraction on the way to separate envelope and tegument fractions. The two fractions were then submitted to immunoblotting with the anti-Flag antibody. Glycoprotein B (gB or UL55) and glycoprotein L (gL or UL115) were also detected by specific antibodies as markers of the envelope fraction. Finally, pp65 (UL83) was chosen as marker of the tegument fraction and the nonstructural HCMV protein IE1 (pp72) to exclude that samples were contaminated by cellular extracts. Results from this analysis are shown in FIG. 3. UL116 was present in the same fraction than gB and completely missing from the tegument fraction in which pp65 was revealed (FIG. 3).

To confirm the presence of pUL116 on the viral envelope, we performed immunoelectron microscopy on the purified TR UL116-Flag purified particles using wt TR viral preparation as control. The 5 nm gold-labeled anti-Flag secondary antibody displayed distinct labeling of the envelope (data not shown). Taken together, these results are consistent with the localization of UL116 on the surface of the HCMV envelope

### Co-transfection and co-immunoprecipitation of recombinant UL116 and glycoprotein H.

To achieve a deeper characterization of the UL116 gene product, we generated a codon optimized recombinant mycHis tagged version of the protein and cloned on a eukaryotic expression vector. Surprisingly, transfection in MRC-5 or HEK293T cells did not result in secretion of the recombinant product or its transport to the cell surface. Confocal analysis of MRC-5 transfected cells showed that UL116 co-localized almost completely with PDI, a marker of the endoplasmic reticulum. We hypothesized that correct localization of UL116 may be achieved following association with one or more of the major HCMV envelope glycoproteins.

To investigate this possibility, first of all we performed binary co-transfection experiments in HEK-293T cells of TR-UL116 with well characterized HCMV envelope glycoproteins (gH, gL and gB and gO). Detection of membrane localization of UL116 was achieved by cytofluorimetric assay on non permeabilized cells with an anti-UL116 mice antisera and a FITC conjugated secondary anti mouse. To exclude possible aspecific detection we included the single transfected populations as negative controls. In FIG. 4A, we show the results from a representative experiment realized with the couples UL116/gH and UL116/gB. FIG. 4A shows a strong UL116 plasma membrane signal only in UL116-gH co-expressing cells (for clarity, gL and gO co-trasfection data are not shown). These data suggest a physical interaction between the gH and UL116 proteins.

To test the formation of a gH-UL116 complex, we performed immunoprecipitation experiments on extracts of HEK293T cells expressing UL116-His plus gH-myc and UL116-His plus gB. As control, single transfection with gH and gB were made. On a single extract, co-immunoprecipitations were performed by anti-His, to detected proteins associated to UL116, and by anti-myc, to reveal species associated to gH. Each immunoprecipitated samples was then divided in three aliquots and treated for Western blot using anti-His (for UL116), anti-myc (for gH) and anti-gB as probes. Results are shown in FIG. 4B. Pulling down UL116, gH resulted associated and not gB (FIG. 5B, bottom panel, lanes 5 and 10 respectively). As countercheck, primary immunoprecipitation of gH by anti-myc resulted in co-immunoprecipitation of UL116 (FIG. 4B middle panel, lane 1). These results showed formation of a gH/UL116 complex using recombinant proteins.

### UL116 localizes in the Assembly Complex and co-immunoprecipitate with gH in infected HFF-1.

Having demonstrated the interaction between recombinant gH and UL116 we sought to confirm these data in the infection system. First we performed co-immunoprecipitation using the anti-gH human monoclonal MSL-109 Ab to pull down gH from TR UL116-Flag infected HFF-1 cell lysates at 96 h p.i. The gH associated proteins were separated on SDS-PAGE and probed in Western blot with an anti-gL as positive control. As negative control a cells lysate from wild-type TR infected HFF-1 was used. Results are shown in FIG. 5. As eluted proteins we found both gL, in both TR-UL116-Flag and wild type infected cells, but also a clear signal for UL116-Flag not present when wt virus was used for infection (FIG. 5).

These data suggest that gH and UL116 are co-transported through the secretory pathway, thus they must also intracellularly co-localize. To further prove this assumption, confocal microscopy was performed on TR UL116-Flag infected HFF-1 cells. TR UL116-Flag infected HFF-1 cells were fixed 96 h p.i. and stained with both anti-gH mAb and anti-Flag mAb. The results revealed an almost complete overlapping of the two proteins restricted to the viral Assembly Complex site. These data demonstrate that UL116 and gH associate during the TR-HCMV replication cycle and traffick to the cellular site of virus assembly and budding.

### Negative stain EM analysis of the purified UL116/gH complex.

We previously used single particles negative stain electron microscopy to characterize gH/gL, gH/gL/gO and Pentamer complexes bound to neutralizing antibodies. Our data revealed that HCMV gH/gL structurally resembles HSV-2 gH/gL and that gO and the UL128/UL130/UL131 subunits bind to the N-terminal end of gH/gL in gH/gL/gO and Pentamer complexes respectively. We also characterized a gL mutant, C144A, that prevents gH/gL homodimerization. Here we extended the EM analysis to the gH/UL116 complex bound to 3G16, a neutralizing Fab binding to the C-terminal domain of gH ((32); Ciferri et al. submitted). The trimeric gH/UL116/3G16-Fab complex was analyzed by single particle electron microscopy. FIG. 7 shows a representative 2D class average of the UL116/gH/3G16 complex in comparison with the gH/gL_{C144A}-3G16 complex (Ciferri et al., submitted). The comparison reveals that the gH/UL116 complex has the same kinked structure observed for the gH/gL complex and that UL116 binds to the N-terminal region of gH on the opposite end of 3G16. Furthermore the EM analysis suggests that UL116 occupies the same site occupied by gL in the gH/gL complexes and that the presence of UL116 does not affect the 3G16 binding site on gH.

### In vivo antigenic properties

An RNA vector expressing both gH and UL116 was generated. Separately, plasmids expressing gH and UL116 were also generated. Recombinantly expressed gH/UL116 complex was purified from HEK293EBNA cell line. Thereafter, using a mouse model, neutralization of infection assays on sera derived from gH/UL116 immunization (both as RNA and protein complex) were performed. CMV complex gH/gL was used as benchmark for immunogenicity assays. The in vivo experiments were carried according to Tables 2 and 3. CNE refers to cationic oil-in-water emulsion (comprising squalene oil, the surfactants Span 85 and Tween 80, and cationic lipid DOTAP). RV01 refers to liposome composition (comprising cationic lipid, zwitterionic lipid, cholesterol, and PEGylated lipid). Both CNE and RV01 were used for delivery of RNA replicons used in this example. Balb-C mice were injected intramuscularly.

**Table 2: study design**

| Gr | N° mice | Vaccination 1 (wk 0, i.m.) | Vaccination 2 (wk 3, i.m.) | Vaccination 3 (wk 6, i.m.) |
|---|---|---|---|---|
| 1 (RNA) | 6 | CNE / gH-gL(Al60) | CNE / gH-gL(Al60) | CNE / gH-gL(Al60) |
| 2 (RNA) | 6 | CNE / gH-UL116 | CNE / gH-UL116 | CNE / gH-UL116 |
| 3 (RNA) | 6 | CNE / gH-gL-UL116 | CNE / gH-gL-UL116 | CNE / gH-gL-UL116 |
| 4 (RNA) | 6 | CNE/UL116 | CNE/UL116 | CNE/UL116 |
| 5 (RNA) | 6 | RV01/gH-gL (A160) | RV01/gH-gL (A160) | RV01/gH-gL (A160) |
| 6 (RNA) | 6 | RV01/gH-UL116 | RV01/gH-UL116 | RV01/gH-UL116 |
| 7 (RNA) | 6 | RV01/gH-gL-UL116 | RV01/gH-hgL-UL116 | RV01/gH-gL-UL116 |
| 8 (RNA) | 6 | RV01/UL116 | RV01/UL116 | RV01/UL116 |
| 9 (protein) | 6 | MF59/gH-UL116 | MF59/gH-UL116 | MF59/gH-UL116 |

**Table 3: immunization and sampling schedules**

| | **Sampling** | **Treatment** |
|---|---|---|
| Day -1 | Pre (pool) | |
| Day 0 | | Immunization 6 mice/group |
| Day 20 | Bleeding (post 1) | |
| Day 21 | | Immunization 6 mice/group |
| Day 41 | Bleeding(post 2) | |
| Day 42 | | Immunization 6 mice/group |
| Day 63 | Bleeding (post 3) | |

Results from these assays (Table 4) show that the gH/UL116 complex display antigenic properties.

**Table 4: Neutralization Titers 50% at 3 week post 3 (day 63)**

| Gr | Vaccination | No. 1 | No. 2 |
|---|---|---|---|
| 1 (RNA) | gH/gL(A160) (15ug/CNE) | 1962 | 927 |
| 2 (RNA) | gH/UL116 (15ug/CNE) | 372 | 303 |
| 3 (RNA) | gH/gL/UL116 (15ug/CNE) | 287 | 181 |
| 4 (RNA) | UL116 (15 ug/CNE) | 100 | 100 |
| 5 (RNA) | gH/gL(A160) (1ug/RV01) | 3077 | 4935 |
| 6 (RNA) | gH/UL116 (1ug/RV01) | 1052 | 928 |
| 7 (RNA) | gH/gL/UL116 (1ug/RV01) | 456 | 2930 |
| 8 (RNA) | UL116 (1ug/RV01) | 100 | 100 |
| 9 (protein) | gH/UL116 (1ug/MF59) | 4407 | 6362 |
| 10 (control) | pre-immune | 100 | 100 |

The various features and embodiments of the present invention, referred to in individual sections above apply, as appropriate, to other sections, mutatis mutandis. Consequently features specified in one section may be combined with features specified in other sections, as appropriate.

The specification is most thoroughly understood in light of the teachings of the references cited within the specification. The embodiments within the specification provide an illustration of embodiments of the invention and should not be construed to limit the scope of the invention. The skilled artisan readily recognizes that many other embodiments are encompassed by the invention. All publications, patents, and GenBank sequences cited in this disclosure are incorporated by reference in their entirety. To the extent the material incorporated by reference contradicts or is inconsistent with this specification, the specification will supersede any such material. The citation of any references herein is not an admission that such references are prior art to the present invention.

Those skilled in the art will recognize, or be able to ascertain using no more than routine experimentation, many equivalents to the specific embodiments of the invention described herein. Such equivalents are intended to be encompassed by the following embodiments.
1. A recombinant human cytomegalovirus (CMV) protein dimeric complex, comprising CMV gH protein or a complex-forming fragment thereof, and CMV UL116 or a complex-forming fragment thereof.
2. The dimeric complex of embodiment 1, wherein said complex-forming fragment of gH does not comprise the signal sequence of a full-length gH protein.
3. The dimeric complex of embodiment 1 or 2, wherein said complex-forming fragment of gH does not comprise the transmembrane domain of a full-length gH protein.
4. The dimeric complex of any one of embodiments 1-3, wherein said complex-forming fragment of gH comprises the ectodomain of a full-length gH protein.
5. The dimeric complex of any one of embodiments 1-4, wherein said complex-forming fragment of gH comprises at least one epitope from SEQ ID NO: 1, SEQ ID NO: 2 , SEQ ID NO: 3, SEQ ID NO: 4, or SEQ ID NO: 5.
6. The dimeric complex of any one of embodiments 1-5, wherein said gH or complex-forming fragment comprises a sequence selected from the group consisting of SEQ ID NOs: 1, 2, 3, 4, and 5.
7. The dimeric complex of any one of embodiments 1-6, wherein said complex-forming fragment of UL116 comprises at least one epitope from SEQ ID NOs: 6, 7, 8, 9, 10, 11, 12, 13, 14, 15, 16, or 17.
8. The dimeric complex of any one of embodiments 1-7, wherein said UL116 or complex-forming fragment comprises a sequence selected from the group consisting of SEQ ID NOs: 6, 7, 8, 9, 10, 11, 12, 13, 14, 15, 16, and 17.
9. The dimeric complex of any one of embodiments 1-8, wherein said gH protein or a complex-forming fragment thereof, and CMV UL116 or a complex-forming fragment thereof, are fused into a single polypeptide chain.
10. An immunogenic composition comprising the dimeric complex of any one of embodiments 1-9.
11. The immunogenic composition of embodiment 10, further comprising an additional CMV protein or CMV protein complex.
12. The immunogenic composition of embodiment 11, wherein said additional CMV protein or CMV protein complex is selected from the group consisting of gB, gH, gL, gO, gM, gN; UL128, UL130, UL131, RL10, RL11, RL12, RL13, UL4, UL5, UL10, UL80.5, UL119, UL122, UL133, UL138, UL148A, UL1, UL7, UL9, UL16, UL18, UL20, UL40, UL41A, UL42, UL47, UL111A, UL124, UL132, UL136, UL141, an immunogenic fragment thereof, a complex-forming fragment thereof, and a combination thereof.
13. The immunogenic composition of embodiment 11 or 12, wherein said additional CMV protein is gB or an immunogenic fragment thereof.
14. The immunogenic composition of any one of embodiments 11-13, wherein said additional CMV protein complex is selected from the group consisting of: gH/gL/UL128/UL130/UL131 pentameric complex, gH/gL complex, gH/gL/gO trimeric complex, gM/gN complex, or a combination thereof.
15. The immunogenic composition of any one of embodiments 10-14, further comprising an adjuvant.
16. The immunogenic composition of embodiment 14, wherein the adjuvant is selected from the group consisting of: an alumimun salt (such as aluminium phosphate, aluminium hydroxide), an oil-in-water emulsion (such as MF59), a TLR7 agonist, IC31, Eisai 57, ISCOM, a CpG oligonucleotide, PET-lipid A, and a combination thereof.
17. An isolated nucleic acid, or a combination of isolated nucleic acids, comprising one or more polynucleotide sequences encoding the dimeric complex of any one of embodiments 1-9.
18. The isolated nucleic acid(s) of embodiment 17, wherein said isolated nucleic acid(s) is RNA, preferably self-replicating RNA.
19. The isolated nucleic acid(s) of embodiment 18, wherein said self-replicating RNA is an alphavirus replicon.
20. An alphavirus replication particle (VRP) comprising the alphavirus replicon of embodiment 19.
21. An immunogenic composition comprising the nucleic acid(s) of any one of embodiments 17-19.
22. An immunogenic composition comprising the VRP of embodiment 20.
23. The immunogenic composition of embodiment 21 or 22, further comprising an adjuvant.
24. The immunogenic composition of embodiment 23, wherein the adjuvant is selected from the group consisting of: an alumimun salt (such as aluminium phosphate, aluminium hydroxide), an oil-in-water emulsion (such as MF59), a TLR7 agonist, IC31, Eisai 57, ISCOM, a CpG oligonucleotide, PET-lipid A, and a combination thereof.
25. A host cell comprising the nucleic acid(s) of any one of embodiments 17-19.
26. The host cell of embodiment 25, wherein said nucleic acid(s) is DNA.
27. The host cell of embodiment 26, wherein said host cell is a mammalian cell.
28. The host cell of embodiment 27, wherein said mammalian cell is a CHO cell or HEK-293 cell.
29. The host cell of any one of embodiments 26-28, wherein said DNA encoding the CMV dimeric complex is stably integrated into the genomic DNA of said host cell.
30. The host cell of any one of embodiments 26-29, wherein, when cultured under a suitable condition, said host cell expresses a soluble gH/UL116 dimeric complex.
31. The host cell of embodiment 30, wherein said soluble dimeric complex is secreted from the host cell.
32. A cell culture comprising the host cell of embodiments 25-31, wherein said culture is at least 20 liter in size.
33. A cell culture comprising the host cell of embodiments 25-31, wherein said culture is at least 100 liter in size.
34. A cell culture comprising the host cell of embodiments 25-31, wherein the yield of said dimeric complex is at least 0.05 g/L.
35. A cell culture comprising the host cell of embodiments 25-31, wherein the yield of said dimeric complex is at least 0.1 g/L.
36. A process of producing a recombinant human cytomegalovirus (CMV) protein dimeric complex, comprising CMV gH protein or a complex-forming fragment thereof, and CMV UL116 or a complex-forming fragment thereof, comprising:
   (i) culturing the host cell of any one of embodiments 25-31 under a suitable condition, thereby expressing said dimeric complex; and
   (ii) harvesting said dimeric complex from the culture.
37. The process of embodiment 36, further comprising purifying said dimeric complex.
38. A recombinant human cytomegalovirus (CMV) protein dimeric complex, comprising CMV gH protein or a complex-forming fragment thereof, and CMV UL116 or a complex-forming fragment thereof, produced by the process of embodiment 36 or 37.
39. A method of inducing an immune response against cytomegalovirus (CMV), comprising administering to a subject in need thereof an immunologically effective amount of the immunogenic composition of any one of embodiments 10-16 and 21-24.
40. The method of embodiment 39, wherein the immune response comprises the production of neutralizing antibodies against CMV.
41. The method of embodiment 40, wherein the neutralizing antibodies are complement-independent.
42. A method of inhibiting cytomegalovirus (CMV) entry into a cell, comprising contacting the cell with the immunogenic composition of any one of embodiments 10-16 and 21-24.
43. The immunogenic composition of any one of embodiments 10-16 and 21-24 for use in inducing an immune response against cytomegalovirus (CMV).
44. Use of the immunogenic composition of any one of embodiments 10-16 and 21-24 for inducing an immune response against cytomegalovirus (CMV).
45. Use of the immunogenic composition of any one of embodiments 10-16 and 21-24 in the manufacture of a medicament for inducing an immune response against cytomegalovirus (CMV).

Appendix: Sequences

SEQ ID NO: 1 (gH from HCMV strain Merlin = GI:52139248)
MRPGLPSYLIIILAVCLFSHLLSSRYGAEAVSEPLDKAFHLLLNTYGRPIRFLRENTTQCTYNSSLRNSTVVRENAIS
FNFFQSYNQYYVFHMPRCLFAGPLAEQFLNQVDLTETLERYQQRLNTYALVSKDLASYRSFSQQLKAQDSLGEQPTT
VPPPIDLSIPHVWMPPQTTPHGWTESHTTSGLHRPHFNQTCILFDGHDLLFSTVTPCLHQGFYLIDELRYVKITLTE
DFFVVTVSIDDDTPMLLIFGHLPRVLFKAPYQRDNFILRQTEKHELLVLVKKDQLNRHSYLKDPDFLDAALDFNYLD
LSALLRNSFHRYAVDVLKSGRCQMLDRRTVEMAFAYALALFAAARQEEAGAQVSVPRALDRQAALLQIQEFMITCLS
QTPPRTTLLLYPTAVDLAKRALWTPNQITDITSLVRLVYILSKQNQQHLIPQWALRQIADFALKLHKTHLASFLSAF
ARQELYLMGSLVHSMLVHTTERREIFIVETGLCSLAELSHFTQLLAHPHHEYLSDLYTPCSSSGRRDHSLERLTRLF
PDATVPATVPAALSILSTMQPSTLETFPDLFCLPLGESFSALTVSEHVSYIVTNQYLIKGISYPVSTTVVGQSLIIT
QTDSQTKCELTRNMHTTHSITVALNISLENCAFCQSALLEYDDTQGVINIMYMHDSDDVLFALDPYNEVVVSSPRTH
YLMLLKNGTVLEVTDVVVDATDSRLLMMSVYALSAIIGIYLLYRMLKTC

SEQ ID NO: 2 (gH from HCMV strain Towne = GI:138314)
MRPGLPSYLIVLAVCLLSHLLSSRYGAEAISEPLDKAFHLLLNTYGRPIRFLRENTTQCTYNSSLRNSTVVRENAIS
FNFFQSYNQYYVFHMPRCLFAGPLAEQFLNQVDLTETLERYQQRLNTYALVSKDLASYRSFSQQLKAQDSLGEQPTT
VPPPIDLSIPHVWMPPQTTPHGWTESHTTSGLHRPHFNQTCILFDGHDLLFSTVTPCLHQGFYLIDELRYVKITLTE
DFFVVTVSIDDDTPMLLIFGHLPRVLFKAPYQRDNFILRQTEKHELLVLVKKDQLNRHSYLKDPDFLDAALDFNYLD
LSALLRNSFHRYAVDVLKSGRCQMLDRRTVEMAFAYALALFAAARQEEAGAQVSVPRALDRQAALLQIQEFMITCLS
QTPPRTTLLLYPTAVDLAKRALWTPNQITDITSLVRLVYILSKQNQQHLIPQWALRQIADFALKLHKTHLASFLSAF
ARQELYLMGSLVHSMLVHTTERREIFIVETGLCSLAELSHFTQLLAHPHHEYLSDLYTPCSSSGRRDHSLERLTRLF
PDATVPTTVPAALSILSTMQPSTLETFPDLFCLPLGESFSALTVSEHVSYVVTNQYLIKGISYPVSTTVVGQSLIIT
QTDSQTKCELTRNMHTTHSITAALNISLENCAFCQSALLEYDDTQGVINIMYMHDSDDVLFALDPYNEVVVSSPRTH
YLMLLKNGTVLEVTDVVVDATDSRLLMMSVYALSAIIGIYLLYRMLKTC

SEQ ID NO: 3 (gH from HCMV strain AD169 = GI:138313)
MRPGLPPYLTVFTVYLLSHLPSQRYGADAASEALDPHAFHLLLNTYGRPIRFLRENTTQCTYNSSLRNSTVVRENAI
SFNFFQSYNQYYVFHMPRCLFAGPLAEQFLNQVDLTETLERYQQRLNTYALVSKDLASYRSFSQQLKAQDSLGQQPT
TVPPPIDLSIPHVWMPPQTTPHDWKGSHTTSGLHRPHFNQTCILFDGHDLLFSTVTPCLHQGFYLMDELRYVKITLT
EDFFVVTVSIDDDTPMLLIFGHLPRVLFKAPYQRDNFILRQTEKHELLVLVKKAQLNRHSYLKDSDFLDAALDFNYL
DLSALLRNSFHRYAVDVLKSGRCQMLDRRTVEMAFAYALALFAAARQEEAGTEISIPRALDRQAALLQIQEFMITCL
SQTPPRTTLLLYPTAVDLAKRALWTPDQITDITSLVRLVYILSKQNQQHLIPQWALRQIADFALQLHKTHLASFLSA
FARQELYLMGSLVHSMLVHTTERREIFIVETGLCSLAELSHFTQLLAHPHHEYLSDLYTPCSSSGRRDHSLERLTRL
FPDATVPATVPAALSILSTMQPSTLETFPDLFCLPLGESFSALTVSEHVSYVVTNQYLIKGISYPVSTTVVGQSLII
TQTDSQTKCELTRNMHTTHSITAALNISLENCAFCQSALLEYDDTQGVINIMYMHDSDDVLFALDPYNEVVVSSPRT
HYLMLLKNGTVLEVTDVVVDATDSRLLMMSVYALSAIIGIYLLYRMLKTC

SEQ ID NO: 4 (gH fragment; amino acid residues 1-715 of SEQ ID NO: 1)
MRPGLPSYLIIILAVCLFSHLLSSRYGAEAVSEPLDKAFHLLLNTYGRPIRFLRENTTQCTYNSSLRNSTVVRENAIS
FNFFQSYNQYYVFHMPRCLFAGPLAEQFLNQVDLTETLERYQQRLNTYALVSKDLASYRSFSQQLKAQDSLGEQPTT
VPPPIDLSIPHVWMPPQTTPHGWTESHTTSGLHRPHFNQTCILFDGHDLLFSTVTPCLHQGFYLIDELRYVKITLTE
DFFVVTVSIDDDTPMLLIFGHLPRVLFKAPYQRDNFILRQTEKHELLVLVKKDQLNRHSYLKDPDFLDAALDFNYLD
LSALLRNSFHRYAVDVLKSGRCQMLDRRTVEMAFAYALALFAAARQEEAGAQVSVPRALDRQAALLQIQEFMITCLS
QTPPRTTLLLYPTAVDLAKRALWTPNQITDITSLVRLVYILSKQNQQHLIPQWALRQIADFALKLHKTHLASFLSAF
ARQELYLMGSLVHSMLVHTTERREIFIVETGLCSLAELSHFTQLLAHPHHEYLSDLYTPCSSSGRRDHSLERLTRLF
PDATVPATVPAALSILSTMQPSTLETFPDLFCLPLGESFSALTVSEHVSYIVTNQYLIKGISYPVSTTVVGQSLIIT
QTDSQTKCELTRNMHTTHSITVALNISLENCAFCQSALLEYDDTQGVINIMYMHDSDDVLFALDPYNEVVVSSPRTH
YLMLLKNGTVLEVTDVVVDATD

SEQ ID NO: 5 (gH fragment; amino acid residues 24-715 of SEQ ID NO: 1)
RYGAEAVSEPLDKAFHLLLNTYGRPIRFLRENTTQCTYNSSLRNSTVVRENAISFNFFQSYNQYYVFHMPRCLFAGP
LAEQFLNQVDLTETLERYQQRLNTYALVSKDLASYRSFSQQLKAQDSLGEQPTTVPPPIDLSIPHVWMPPQTTPHGW
TESHTTSGLHRPHFNQTCILFDGHDLLFSTVTPCLHQGFYLIDELRYVKITLTEDFFVVTVSIDDDTPMLLIFGHLP
RVLFKAPYQRDNFILRQTEKHELLVLVKKDQLNRHSYLKDPDFLDAALDFNYLDLSALLRNSFHRYAVDVLKSGRCQ
MLDRRTVEMAFAYALALFAAARQEEAGAQVSVPRALDRQAALLQIQEFMITCLSQTPPRTTLLLYPTAVDLAKRALW
TPNQITDITSLVRLVYILSKQNQQHLIPQWALRQIADFALKLHKTHLASFLSAFARQELYLMGSLVHSMLVHTTERR

EIFIVETGLCSLAELSHFTQLLAHPHHEYLSDLYTPCSSSGRRDHSLERLTRLFPDATVPATVPAALSILSTMQPST
LETFPDLFCLPLGESFSALTVSEHVSYIVTNQYLIKGISYPVSTTVVGQSLIITQTDSQTKCELTRNMHTTHSITVA
LNISLENCAFCQSALLEYDDTQGVINIMYMHDSDDVLFALDPYNEVVVSSPRTHYLMLLKNGTVLEVTDVVVDATD

SEQ ID NOs. 6-17 (Alignment of UL116 proteins from different CMV strains)

Merlin    1 MKRRRRWRGWLLFLALCFCLLCEAVETNATTVTSTTAAAATTNTTVATTGTTTTSPNVTS
TR-BAC    1 MKRRRRWRGWLLFLALCFCLLCEAVETNATTVTGTTAAAATTNTTVATTGTTTTSPNVTS
VR1814    1 MKRRRRWRGWLLFLALCFCLLCEAVETNATTVTSTTAAAATTNTTVATTGTTTTSPNVTS
Towne     1 MKRRRRWRGWLLFLALCFCLLCEAVETNATTVTSTTAAAATTNTTVATTGTTTTSPNVTS
AD169     1 MKRRRRWRGWLLFPALCFCLLCEAVETNATTVTSTTAAAATTNTTVATTGTTTTSPNVTS
TB40/E    1 MKRRRRWRGWLLFLALCFCLLCEAVETNATTVTSTTAAAATTNTTVATTGTTTTSPNVTS
HAN38     1 MKRRRRWRGWLLFLALCFCLLCEAVETNATTVTSTTAAAATTNTTVATTGTTTTSPNVTS
3301      1 MKRRRRWRGWLLFLALCFCLLCEAVETNTTTVTSTTAAAATTNTTVATTGTTTTSPNVTS
U8        1 MKRRRRWRGWLLFLALCFCLLCEAVETNMTTVTSTTAAAATTNTTVATTGTTTTSPNVTS
HAN20     1 MKRRRRWRGWLLFLALCFCLLCEAVETNATTVTSTTAAAATTNTTVATTGTTTTSPNVTS
3157      1 MKRRRRWRGWLLFLALCFCLLREAVETNTTTVTSTTAAAATTNTTVATTGTTTTSPNVTS
HAN13     1 MKRRRRWRGWLLFLALCFCLLREAVETNTTTVTSTTAAAATTNTTVATTGTTTTSPNVTS

Merlin   61 TTSNTVTTPTTVSSVSNLTSSATSILISTSTVSGTRNTRNNNTTTIGTNATSPSSSVSIL
TR-BAC   61 TTSNTVTTPTTVSSVSNLTSSTTSIPISTSTVSGTRNTGNNNTTTIGTNATSPSPSVSIL
VR1814   61 TTSNTVITPTTVSSVSNLTSSATSIPISTSTVSGTRNTRNNNTTTIGTNVTSPSPSVSIL
Towne    61 TTSNTVTTPTTVSSVSNLTSSATSIPISTSTVSETRNTRNNNTTTIGTNATSPSPSVSIL
AD169    61 TTSNTVTTPTTVSSVSNLTSSTTSIPISTSTVSGTRNTGNNNTTTIGTNATSPSPSVSIL
TB40/E   61 TTSNTVITPTTVSSVSNLTSSATSIPISTSTVSGTRNTRNNNTTTIGTNVTSPSPSVSIL
HAN38    61 TTSNTVTTPTTVSSVSNLTSSATSIPISTSTVSETRNTRNNNTTTIGTNATSPSPSVSIL
3301     61 TTSNTVTTPTTVSSVSNLTSSTTSIPISTSTVSGTRNTGNNNTTTIGTNATSPSPSVSIL
U8       61 TTSNTITTPTTVSSVSNLTSSTTSIPISTSTVSGTRNTGNNNTTTIGTNATSPSPSVSIL
HAN20    61 TTSNTVTTPTTVSSVSNLTSNTTSIPISTSTVSGTKSTGNNNTTTIGTNATSPSPSVSIL
3157     61 TTSNTVTTPTTVSSVSNLTSSATSIPISTSTVSGIRNTGNNNTTTIGTNATSPSPSVSIL
HAN13    61 TTSNTVTTPTTVSSVSNLTSSATSIPISTSTVSGIRNTGNNNTTTIGTNATSPSPSVSIL

Merlin  121 TTVTPAATSTTSNNGDVTSDYTPTFDLENITTTRAPTRPPAQDLCSHNLSIILYEEESQS
TR-BAC  121 TTATPAATSTTSNNGDVTSDYTPTFDLENITTTRAPTRPPAQDLCSHNLSIILYEEESQS
VR1814  121 TTVTPAATSTTSNNGDVTSDYTPTFDLENITTTRAPTRPPAQDLCSHNLSIILYEEESQS
Towne   121 TTVTPAATSTISVDGVVTASDYTPTFDDLENITTTRAPTRPPAQDLCSHNLSIILYEEES
AD169   121 TTVTPAATSTISVDGVVTASDYTPTFDDLENITTTRAPTRPPAQDLCSHNLSIILYEEES
TB40/E  121 TTVTPAATSTTSNNGDVTSDYTPTFDLENITTTRAPTRPPAQDLCSHNLSIILYEEESQS
HAN38   121 TTVTPAATSTTSNNGDVTSDYTPTFDLENITTTRAPTRPPAQDLCSHNLSIILYEEESQS
3301    121 TTVTPAATSTTSNNGDVTSDYTPTFDLENITTTRAPTRPPAQDLCSHNLSIILYEEESQS
U8      121 TTVTPAATSTTSNNGDVTSDYTPTFDLENITTTRAPTRPPAQDLCSHNLSIILYEEESQS
HAN20   121 TTVTPAATSTISVDGVVTTSDYTPTFDDLENITTTRAPTRPPAQDLCSHNLSIILYEEES
3157    121 TTVTPAATSTTSNNGDVTSDYTPTFDLENITTTRAPTRPPAQDLCSHNLSIILYEEESQS
HAN13   121 TTVTPAATSTTSNNGDVTSDYTPTFDLENITTTRAPTRPPAQDLCSHNLSIILYEEESQS

Merlin  181 SVDIAVDEEEPELEDDDEYDELWFPLYFEAECNLNYTLQYVNHSCDYSVRQSSVSFPPWR
TR-BAC  181 SVDIAVDEEEPELEDDDEYDELWFPLYFEAECNLNYTLQYVNHSCDYSVRQSSVSFPPWR
VR1814  181 SVDIAVDEEEPELEDDDEYDELWFPLYFEAECNLNYTLQYVNHSCDYSVRQSSVSFPPWR
Towne   181 QSSVDIAVDEEEPELEDDDEYDELWFPLYFEAECNLNYTLQYVNHSCDYSVRQSSVSFPP
AD169   181 QSSVDIAVDEEEPELEDDDEYDELWFPLYFEAECNRNYTLHVNHSCDYSVRQSSVSFPPW
TB40/E  181 SVDIAVDEEEPELEDDDEYDELWFPLYFEAECNLNYTLQYVNHSCDYSVRQSSVSFPPWR
HAN38   181 SVDIAVDEEEPELEDDDEYDELWFPLYFEAECNLNYTLQYVNHSCDYSVRQSSVSFPPWR
3301    181 SVDITVGEEESESEEDDDEEYDELWFPLYFEAECNLNYTLQYVNHSCDYSVRQSSVSFPP
U8      181 SVDITVGEEESESEEDDDEEYDELWFPLYFEAECNLNYTLQYVNHSCDYSVRQSSVSFPP

HAN20    181 QSSVDIAVDEEEPELEDDDEYDELWFPLYFEAECNLNYTLQYVNHSCDYSVRQSSVSFPP
3157     181 SVDITVGEEESESEEDDDEEYDELWFPLYFEAECNLNYTLQYVNHSCDYSVRQSSVSFPP
HAN13    181 SVDITVGEEESELEEDDDEEYDELWFPLYFEAECNRNYTLHVNHSCDYSVRQSSVSFPPW

Merlin   241 DIDSVTFVPRNLSNCSAHGLAVIVAGNQTWYVNPFSLAHLLDAIYNVLGIEDLSANFWRQ
TR-BAC   241 DIDSVTFVPRNLSNCSAHGLAVIVAGNQTWYVNPFSLAHLLDAIYNVLGIEDLSANFRRQ
VR1814   241 DIDSVTFVPRNLSNCSAHGLAVIVAGNQTWYVNPFSLAHLLDAIYNVLGIEDLSANFRRQ
Towne    241 WRDIDSVTFVPRNLSNCSAHGLAVIVAGNQTWYVNPFSLAHLLDAIYNVLGIEDLSANFR
AD169    241 RDIDSVTFVPRNLSNCSAHGLAVIVAGNQTWYVNPFSLAHLLDAIYNVLGIEDLSANFRR
TB40/E   241 DIDSVTFVPRNLSNCSAHGLAVIVAGNQTWYVNPFSLAHLLDAIYNVLGIEDLSANFRRQ
HAN38    241 DIDSVTFVPRNLSNCSAHGLAVIVAGNQTWYVNPFSLAHLLDAIYNVLGIEDLSANFRRQ
3301     241 WRDIDSVTFVPRNLSNCSAHGLAVIVAGNQTWYVNPFSLAHLLDAIYNVLGIEDLSANFR
U8       241 WRDIDSVTFVPRNLSNCSAHGLAVIVAGNQTWYVNPFSLAHLLDAIYNVLGIEDLSANFR
HAN20    241 WRDIDSVTFVPRNLSNCSAHGLAVIVAGNQTWYVNPFSLAHLLDAIYNVLGIEDLSANFR
3157     241 WRDIDSVTFVPRNLSNCSAHGLAVIVAGNQTWYVNPFSLAHLLDAIYNVLGIEDLSANFR
HAN13    241 RDIDSVTFVPRNLSNCSAHGLAVIVAGNQTWYVNPFSLAHLLDAIYNVLGIEDLSANFRR

UL116 protein from Neut strain:

TB40/e-UL32-GFP, DNA:

ATGAAGCGGCGGCGGCGATGGCGGGGCTGGTTGCTTTTCCTGGCCCTGTGCTTTTGCTTACTGTGTGAAGCGGTGGA
AACCAACGCGACCACCGTTACCAGTACCACCGCTGCCGCCGCCACGACAAACACTACCGTCGCCACCACCGGTACCA
CTACTACCTCCCCTAACGTCACTTCAACCACGAGTAACACCGTCATCACTCCCACCACGGTTTCCTCGGTCAGCAAT
CTGACATCCAGCGCCACGTCGATTCCCATCTCAACGTCAACGGTTTCTGGAACAAGAAACACAAGGAATAATAATAC
CACAACCATCGGTACGAACGTTACTTCCCCCTCCCCTTCTGTATCCATACTTACCACCGTGACACCGGCCGCGACTT
CTACCACCTCCAACAACGGGGATGTAACATCCGACTACACTCCAACTTTTGACCTGGAAAACATTACCACCACCCGC
GCTCCCACGCGTCCTCCCGCCCAGGACCTTTGTAGCCATAACCTGTCAATCATCCTGTACGAAGAGGAATCTCAGAG
CAGCGTAGACATTGCGGTGGATGAAGAAGAGCCAGAACTGGAGGACGACGACGAGTACGACGAACTGTGGTTCCCCC
TCTACTTCGAGGCTGAGTGCAACCTAAATTACACGCTACAATACGTCAATCACAGTTGTGATTACAGCGTGCGCCAG
TCGTCTGTCTCATTCCCCCCGTGGCGCGACATCGACTCAGTTACCTTCGTACCCAGGAACCTCTCCAACTGTAGCGC
CCACGGTCTGGCCGTCATCGTCGCGGGTAACCAAACCTGGTACGTGAATCCGTTTAGCCTGGCTCACCTGCTGGATG
CAATATATAACGTTTTAGGGATCGAAGACCTGAGCGCCAACTTTCGGCGCCAACTGGCTCCTTACCGTCACACTCTC
ATCGTGCCGCAGACT (SEQ ID NO:24)

TB40/e-UL32-GFP, Protein:

MKRRRRWRGWLLFLALCFCLLCEAVETNATTVTSTTAAAATTNTTVATTGTTTTSPNVTSTTSNTVITPTTVSSVSN
LTSSATSIPISTSTVSGTRNTRNNNTTTIGTNVTSPSPSVSILTTVTPAATSTTSNNGDVTSDYTPTFDLENITTTR
APTRPPAQDLCSHNLSIILYEEESQSSVDIAVDEEEPELEDDDEYDELWFPLYFEAECNLNYTLQYVNHSCDYSVRQ
SSVSFPPWRDIDSVTFVPRNLSNCSAHGLAVIVAGNQTWYVNPFSLAHLLDAIYNVLGIEDLSANFRRQLAPYRHTL
IVPQT (SEQ ID NO: 25)

UL116 from Immunization strain:

TR, DNA (codon-optimized*):

ATGAAGCGGCGGAGAAGATGGCGGGGCTGGCTGCTGTTCCTGGCCCTGTGCTTCTGTCTGCTGTGCGAGGCCGTGGA
GACAAACGCCACCACCGTGACCGGAACAACAGCCGCCGCTGCCACCACCAATACCACTGTCGCCACCACCGGCACCA
CCACCACCTCCCCCAACGTGACCAGCACCACAAGCAACACCGTGACCACCCCTACCACCGTGTCCAGCGTGTCCAAC
CTGACCTCCAGCACAACCTCCATCCCCATCAGCACCAGCACCGTGTCCGGCACCCGGAACACCGGCAACAACAATAC
CACCACCATCGGGACTAACGCTACCTCTCCCAGCCCTTCCGTGAGCATCCTGACCACAGCCACCCCAGCCGCTACCT
CCACAACCAGCAACAACGGCGACGTGACCTCCGACTACACCCCCACCTTCGACCTGGAAAACATCACCACCACAAGA
GCCCCTACCAGACCCCCTGCCCAGGATCTGTGCAGCCACAACCTGAGCATCATCCTGTACGAGGAAGAGTCCCAGAG
CAGCGTGGATATCGCCGTGGACGAGGAAGAACCCGAGCTGGAAGATGACGACGAGTACGACGAGCTGTGGTTCCCCC

TGTACTTCGAGGCCGAGTGCAACCTGAACTACACCCTGCAGTACGTGAACCACAGCTGCGACTACAGCGTGCGGCAG
TCCTCCGTGAGCTTCCCCCCCTGGCGGGACATCGACAGCGTGACCTTCGTGCCCCGGAACCTGAGCAATTGCAGCGC
CCACGGCCTGGCTGTGATCGTGGCCGGCAACCAGACTTGGTACGTGAATCCCTTCAGCCTGGCCCACCTGCTGGACG
CCATCTACAACGTGCTGGGCATCGAGGACCTGAGCGCCAACTTCAGACGGCAGCTGGCCCCCTACAGACACACCCTG
ATCGTGCCCCAGACC (SEQ ID NO: 26)

Protein:

MKRRRRWRGWLLFLALCFCLLCEAVETNATTVTGTTAAAATTNTTVATTGTTTTSPNVTSTTSNTVTTPTTVSSVSN
LTSSTTSIPISTSTVSGTRNTGNNNTTTIGTNATSPSPSVSILTTATPAATSTTSNNGDVTSDYTPTFDLENITTTR
APTRPPAQDLCSHNLSIILYEEESQSSVDIAVDEEEPELEDDDEYDELWFPLYFEAECNLNYTLQYVNHSCDYSVRQ
SSVSFPPWRDIDSVTFVPRNLSNCSAHGLAVIVAGNQTWYVNPFSLAHLLDAIYNVLGIEDLSANFRRQLAPYRHTL
IVPQT (SEQ ID NO: 27)

## Claims

1. A recombinant human cytomegalovirus (CMV) protein dimeric complex, comprising CMV gH protein or a complex-forming fragment thereof, and CMV UL116 or a complex-forming fragment thereof.

2. The dimeric complex of claim 1, wherein said complex-forming fragment of gH does not comprise the transmembrane domain of a full-length gH protein.

3. The dimeric complex of claim 1 or 2, wherein said complex-forming fragment of gH comprises the ectodomain of a full-length gH protein.

4. The dimeric complex of any one of claims 1-3, wherein said gH or complex-forming fragment comprises a sequence selected from the group consisting of SEQ ID NOs: 1, 2, 3, 4, and 5.

5. The dimeric complex of any one of claims 1-4, wherein said UL116 or complex-forming fragment comprises a sequence selected from the group consisting of SEQ ID NOs: 6, 7, 8, 9, 10, 11, 12, 13, 14, 15, 16, and 17.

6. The dimeric complex of any one of claims 1-5, wherein said gH protein or a complex-forming fragment thereof, and CMV UL116 or a complex-forming fragment thereof, are fused into a single polypeptide chain.

7. An immunogenic composition comprising the dimeric complex of any one of claims 1-6, optionally comprising an adjuvant.

8. The immunogenic composition of claim 7, further comprising an additional CMV protein or CMV protein complex.

9. The immunogenic composition of claim 8, wherein said additional CMV protein is gB or an immunogenic fragment thereof.

10. The immunogenic composition of claim 8 or 9, wherein said additional CMV protein complex is selected from the group consisting of: gH/gL/UL128/UL130/UL131 pentameric complex, gH/gL complex, gH/gL/gO trimeric complex, gM/gN complex, or a combination thereof.

11. An isolated nucleic acid, or a combination of isolated nucleic acids, comprising one or more polynucleotide sequences encoding the dimeric complex of any one of claims 1-6.

12. The isolated nucleic acid(s) of claim 11, wherein said isolated nucleic acid(s) is RNA, preferably self-replicating RNA.

13. An immunogenic composition comprising the nucleic acid(s) of claim 11 or 12, optionally comprising an adjuvant.

14. A host cell comprising the nucleic acid(s) of claim 11 or 12.

15. The immunogenic composition of any one of claims 7-10 and 13, for use in inducing an immune response against cytomegalovirus (CMV).


SEQUENCE LISTING

<110> NOVARTIS AG

<120> CMV ANTIGEN AND USES THEREOF

<130> PAT056493-EP-EPA

<140>
<141>

<160> 27

<170> PatentIn version 3.5

<210> 1
<211> 742
<212> PRT
<213> Human herpesvirus 5

<400> 1
   Met Arg Pro Gly Leu Pro Ser Tyr Leu Ile Ile Leu Ala Val Cys Leu
   1               5                   10                  15
   Phe Ser His Leu Leu Ser Ser Arg Tyr Gly Ala Glu Ala Val Ser Glu
               20                  25                  30
   Pro Leu Asp Lys Ala Phe His Leu Leu Leu Asn Thr Tyr Gly Arg Pro
           35                  40                  45
   Ile Arg Phe Leu Arg Glu Asn Thr Thr Gln Cys Thr Tyr Asn Ser Ser
       50                  55                  60
   Leu Arg Asn Ser Thr Val Val Arg Glu Asn Ala Ile Ser Phe Asn Phe
   65                  70                  75                  80
   Phe Gln Ser Tyr Asn Gln Tyr Tyr Val Phe His Met Pro Arg Cys Leu
                   85                  90                  95
   Phe Ala Gly Pro Leu Ala Glu Gln Phe Leu Asn Gln Val Asp Leu Thr
               100                 105                 110
   Glu Thr Leu Glu Arg Tyr Gln Gln Arg Leu Asn Thr Tyr Ala Leu Val
           115                 120                 125
   Ser Lys Asp Leu Ala Ser Tyr Arg Ser Phe Ser Gln Gln Leu Lys Ala
       130                 135                 140
   Gln Asp Ser Leu Gly Glu Gln Pro Thr Thr Val Pro Pro Pro Ile Asp
   145                 150                 155                 160
   Leu Ser Ile Pro His Val Trp Met Pro Pro Gln Thr Thr Pro His Gly
                   165                 170                 175
   Trp Thr Glu Ser His Thr Thr Ser Gly Leu His Arg Pro His Phe Asn
           180                 185                 190
   Gln Thr Cys Ile Leu Phe Asp Gly His Asp Leu Leu Phe Ser Thr Val
       195                 200                 205
   Thr Pro Cys Leu His Gln Gly Phe Tyr Leu Ile Asp Glu Leu Arg Tyr
       210                 215                 220
   Val Lys Ile Thr Leu Thr Glu Asp Phe Phe Val Val Thr Val Ser Ile
   225                 230                 235                 240
   Asp Asp Asp Thr Pro Met Leu Leu Ile Phe Gly His Leu Pro Arg Val
                   245                 250                 255
   Leu Phe Lys Ala Pro Tyr Gln Arg Asp Asn Phe Ile Leu Arg Gln Thr
           260                 265                 270
   Glu Lys His Glu Leu Leu Val Leu Val Lys Lys Asp Gln Leu Asn Arg
       275                 280                 285
   His Ser Tyr Leu Lys Asp Pro Asp Phe Leu Asp Ala Ala Leu Asp Phe
       290                 295                 300
   Asn Tyr Leu Asp Leu Ser Ala Leu Leu Arg Asn Ser Phe His Arg Tyr
   305                 310                 315                 320
   Ala Val Asp Val Leu Lys Ser Gly Arg Cys Gln Met Leu Asp Arg Arg
                   325                 330                 335
   Thr Val Glu Met Ala Phe Ala Tyr Ala Leu Ala Leu Phe Ala Ala Ala
           340                 345                 350
   Arg Gln Glu Glu Ala Gly Ala Gln Val Ser Val Pro Arg Ala Leu Asp
       355                 360                 365
   Arg Gln Ala Ala Leu Leu Gln Ile Gln Glu Phe Met Ile Thr Cys Leu
       370                 375                 380
   Ser Gln Thr Pro Pro Arg Thr Thr Leu Leu Leu Tyr Pro Thr Ala Val
   385                 390                 395                 400
   Asp Leu Ala Lys Arg Ala Leu Trp Thr Pro Asn Gln Ile Thr Asp Ile
                   405                 410                 415
   Thr Ser Leu Val Arg Leu Val Tyr Ile Leu Ser Lys Gln Asn Gln Gln
   420 425 430
   His Leu Ile Pro Gln Trp Ala Leu Arg Gln Ile Ala Asp Phe Ala Leu
   435 440 445
   Lys Leu His Lys Thr His Leu Ala Ser Phe Leu Ser Ala Phe Ala Arg
   450 455 460
   Gln Glu Leu Tyr Leu Met Gly Ser Leu Val His Ser Met Leu Val His
   465 470 475 480
   Thr Thr Glu Arg Arg Glu Ile Phe Ile Val Glu Thr Gly Leu Cys Ser
   485 490 495
   Leu Ala Glu Leu Ser His Phe Thr Gln Leu Leu Ala His Pro His His
   500 505 510
   Glu Tyr Leu Ser Asp Leu Tyr Thr Pro Cys Ser Ser Ser Gly Arg Arg
   515 520 525
   Asp His Ser Leu Glu Arg Leu Thr Arg Leu Phe Pro Asp Ala Thr Val
   530 535 540
   Pro Ala Thr Val Pro Ala Ala Leu Ser Ile Leu Ser Thr Met Gln Pro
   545 550 555 560
   Ser Thr Leu Glu Thr Phe Pro Asp Leu Phe Cys Leu Pro Leu Gly Glu
   565 570 575
   Ser Phe Ser Ala Leu Thr Val Ser Glu His Val Ser Tyr Ile Val Thr
   580 585 590
   Asn Gln Tyr Leu Ile Lys Gly Ile Ser Tyr Pro Val Ser Thr Thr Val
   595 600 605
   Val Gly Gln Ser Leu Ile Ile Thr Gln Thr Asp Ser Gln Thr Lys Cys
   610 615 620
   Glu Leu Thr Arg Asn Met His Thr Thr His Ser Ile Thr Val Ala Leu
   625 630 635 640
   Asn Ile Ser Leu Glu Asn Cys Ala Phe Cys Gln Ser Ala Leu Leu Glu
   645 650 655
   Tyr Asp Asp Thr Gln Gly Val Ile Asn Ile Met Tyr Met His Asp Ser
   660 665 670
   Asp Asp Val Leu Phe Ala Leu Asp Pro Tyr Asn Glu Val Val Val Ser
           675                 680                 685
   Ser Pro Arg Thr His Tyr Leu Met Leu Leu Lys Asn Gly Thr Val Leu
       690                 695                 700
   Glu Val Thr Asp Val Val Val Asp Ala Thr Asp Ser Arg Leu Leu Met
   705                 710                 715                 720
   Met Ser Val Tyr Ala Leu Ser Ala Ile Ile Gly Ile Tyr Leu Leu Tyr
                   725                 730                 735
   Arg Met Leu Lys Thr Cys
               740

<210> 2
<211> 742
<212> PRT
<213> Human herpesvirus 5

<400> 2
   Met Arg Pro Gly Leu Pro Ser Tyr Leu Ile Val Leu Ala Val Cys Leu
   1               5                   10                  15
   Leu Ser His Leu Leu Ser Ser Arg Tyr Gly Ala Glu Ala Ile Ser Glu
               20                  25                  30
   Pro Leu Asp Lys Ala Phe His Leu Leu Leu Asn Thr Tyr Gly Arg Pro
           35                  40                  45
   Ile Arg Phe Leu Arg Glu Asn Thr Thr Gln Cys Thr Tyr Asn Ser Ser
       50                  55                  60
   Leu Arg Asn Ser Thr Val Val Arg Glu Asn Ala Ile Ser Phe Asn Phe
   65                  70                  75                  80
   Phe Gln Ser Tyr Asn Gln Tyr Tyr Val Phe His Met Pro Arg Cys Leu
                   85                  90                  95
   Phe Ala Gly Pro Leu Ala Glu Gln Phe Leu Asn Gln Val Asp Leu Thr
               100                 105                 110
   Glu Thr Leu Glu Arg Tyr Gln Gln Arg Leu Asn Thr Tyr Ala Leu Val
           115                 120                 125
   Ser Lys Asp Leu Ala Ser Tyr Arg Ser Phe Ser Gln Gln Leu Lys Ala
       130                 135                 140
   Gln Asp Ser Leu Gly Glu Gln Pro Thr Thr Val Pro Pro Pro Ile Asp
   145                 150                 155                 160
   Leu Ser Ile Pro His Val Trp Met Pro Pro Gln Thr Thr Pro His Gly
                   165                 170                 175
   Trp Thr Glu Ser His Thr Thr Ser Gly Leu His Arg Pro His Phe Asn
               180                 185                 190
   Gln Thr Cys Ile Leu Phe Asp Gly His Asp Leu Leu Phe Ser Thr Val
           195                 200                 205
   Thr Pro Cys Leu His Gln Gly Phe Tyr Leu Ile Asp Glu Leu Arg Tyr
       210                 215                 220
   Val Lys Ile Thr Leu Thr Glu Asp Phe Phe Val Val Thr Val Ser Ile
   225                 230                 235                 240
   Asp Asp Asp Thr Pro Met Leu Leu Ile Phe Gly His Leu Pro Arg Val
                   245                 250                 255
   Leu Phe Lys Ala Pro Tyr Gln Arg Asp Asn Phe Ile Leu Arg Gln Thr
               260                 265                 270
   Glu Lys His Glu Leu Leu Val Leu Val Lys Lys Asp Gln Leu Asn Arg
           275                 280                 285
   His Ser Tyr Leu Lys Asp Pro Asp Phe Leu Asp Ala Ala Leu Asp Phe
       290                 295                 300
   Asn Tyr Leu Asp Leu Ser Ala Leu Leu Arg Asn Ser Phe His Arg Tyr
   305                 310                 315                 320
   Ala Val Asp Val Leu Lys Ser Gly Arg Cys Gln Met Leu Asp Arg Arg
                   325                 330                 335
   Thr Val Glu Met Ala Phe Ala Tyr Ala Leu Ala Leu Phe Ala Ala Ala
               340                 345                 350
   Arg Gln Glu Glu Ala Gly Ala Gln Val Ser Val Pro Arg Ala Leu Asp
           355                 360                 365
   Arg Gln Ala Ala Leu Leu Gln Ile Gln Glu Phe Met Ile Thr Cys Leu
       370                 375                 380
   Ser Gln Thr Pro Pro Arg Thr Thr Leu Leu Leu Tyr Pro Thr Ala Val
   385                 390                 395                 400
   Asp Leu Ala Lys Arg Ala Leu Trp Thr Pro Asn Gln Ile Thr Asp Ile
                   405                 410                 415
   Thr Ser Leu Val Arg Leu Val Tyr Ile Leu Ser Lys Gln Asn Gln Gln
               420                 425                 430
   His Leu Ile Pro Gln Trp Ala Leu Arg Gln Ile Ala Asp Phe Ala Leu
           435                 440                 445
   Lys Leu His Lys Thr His Leu Ala Ser Phe Leu Ser Ala Phe Ala Arg
       450                 455                 460
   Gln Glu Leu Tyr Leu Met Gly Ser Leu Val His Ser Met Leu Val His
   465                 470                 475                 480
   Thr Thr Glu Arg Arg Glu Ile Phe Ile Val Glu Thr Gly Leu Cys Ser
                   485                 490                 495
   Leu Ala Glu Leu Ser His Phe Thr Gln Leu Leu Ala His Pro His His
               500                 505                 510
   Glu Tyr Leu Ser Asp Leu Tyr Thr Pro Cys Ser Ser Ser Gly Arg Arg
           515                 520                 525
   Asp His Ser Leu Glu Arg Leu Thr Arg Leu Phe Pro Asp Ala Thr Val
       530                 535                 540
   Pro Thr Thr Val Pro Ala Ala Leu Ser Ile Leu Ser Thr Met Gln Pro
   545                 550                 555                 560
   Ser Thr Leu Glu Thr Phe Pro Asp Leu Phe Cys Leu Pro Leu Gly Glu
                   565                 570                 575
   Ser Phe Ser Ala Leu Thr Val Ser Glu His Val Ser Tyr Val Val Thr
               580                 585                 590
   Asn Gln Tyr Leu Ile Lys Gly Ile Ser Tyr Pro Val Ser Thr Thr Val
           595                 600                 605
   Val Gly Gln Ser Leu Ile Ile Thr Gln Thr Asp Ser Gln Thr Lys Cys
       610                 615                 620
   Glu Leu Thr Arg Asn Met His Thr Thr His Ser Ile Thr Ala Ala Leu
   625                 630                 635                 640
   Asn Ile Ser Leu Glu Asn Cys Ala Phe Cys Gln Ser Ala Leu Leu Glu
                   645                 650                 655
   Tyr Asp Asp Thr Gln Gly Val Ile Asn Ile Met Tyr Met His Asp Ser
               660                 665                 670
   Asp Asp Val Leu Phe Ala Leu Asp Pro Tyr Asn Glu Val Val Val Ser
           675                 680                 685
   Ser Pro Arg Thr His Tyr Leu Met Leu Leu Lys Asn Gly Thr Val Leu
       690                 695                 700
   Glu Val Thr Asp Val Val Val Asp Ala Thr Asp Ser Arg Leu Leu Met
   705                 710                 715                 720
   Met Ser Val Tyr Ala Leu Ser Ala Ile Ile Gly Ile Tyr Leu Leu Tyr
                   725                 730                 735
   Arg Met Leu Lys Thr Cys
               740

<210> 3
<211> 743
<212> PRT
<213> Human herpesvirus 5

<400> 3
Met Arg Pro Gly Leu Pro Pro Tyr Leu Thr Val Phe Thr Val Tyr Leu
1               5                   10                  15

Leu Ser His Leu Pro Ser Gln Arg Tyr Gly Ala Asp Ala Ala Ser Glu
            20                  25                  30

Ala Leu Asp Pro His Ala Phe His Leu Leu Leu Asn Thr Tyr Gly Arg
        35                  40                  45

Pro Ile Arg Phe Leu Arg Glu Asn Thr Thr Gln Cys Thr Tyr Asn Ser
    50                  55                  60

Ser Leu Arg Asn Ser Thr Val Val Arg Glu Asn Ala Ile Ser Phe Asn
65                  70                  75                  80

Phe Phe Gln Ser Tyr Asn Gln Tyr Tyr Val Phe His Met Pro Arg Cys
                85                  90                  95

Leu Phe Ala Gly Pro Leu Ala Glu Gln Phe Leu Asn Gln Val Asp Leu
            100                 105                 110

Thr Glu Thr Leu Glu Arg Tyr Gln Gln Arg Leu Asn Thr Tyr Ala Leu
        115                 120                 125

Val Ser Lys Asp Leu Ala Ser Tyr Arg Ser Phe Ser Gln Gln Leu Lys
    130                 135                 140

Ala Gln Asp Ser Leu Gly Gln Gln Pro Thr Thr Val Pro Pro Pro Ile
145                 150                 155                 160

Asp Leu Ser Ile Pro His Val Trp Met Pro Pro Gln Thr Thr Pro His
                165                 170                 175

Asp Trp Lys Gly Ser His Thr Thr Ser Gly Leu His Arg Pro His Phe
            180                 185                 190

Asn Gln Thr Cys Ile Leu Phe Asp Gly His Asp Leu Leu Phe Ser Thr
        195                 200                 205

Val Thr Pro Cys Leu His Gln Gly Phe Tyr Leu Met Asp Glu Leu Arg
    210                 215                 220

Tyr Val Lys Ile Thr Leu Thr Glu Asp Phe Phe Val Val Thr Val Ser
225                 230                 235                 240

Ile Asp Asp Asp Thr Pro Met Leu Leu Ile Phe Gly His Leu Pro Arg
                245                 250                 255

Val Leu Phe Lys Ala Pro Tyr Gln Arg Asp Asn Phe Ile Leu Arg Gln
            260                 265                 270

Thr Glu Lys His Glu Leu Leu Val Leu Val Lys Lys Ala Gln Leu Asn
        275                 280                 285

Arg His Ser Tyr Leu Lys Asp Ser Asp Phe Leu Asp Ala Ala Leu Asp
    290                 295                 300

Phe Asn Tyr Leu Asp Leu Ser Ala Leu Leu Arg Asn Ser Phe His Arg
305                 310                 315                 320

Tyr Ala Val Asp Val Leu Lys Ser Gly Arg Cys Gln Met Leu Asp Arg
                325                 330                 335

Arg Thr Val Glu Met Ala Phe Ala Tyr Ala Leu Ala Leu Phe Ala Ala
            340                 345                 350

Ala Arg Gln Glu Glu Ala Gly Thr Glu Ile Ser Ile Pro Arg Ala Leu
        355                 360                 365

Asp Arg Gln Ala Ala Leu Leu Gln Ile Gln Glu Phe Met Ile Thr Cys
    370                 375                 380

Leu Ser Gln Thr Pro Pro Arg Thr Thr Leu Leu Leu Tyr Pro Thr Ala
385                 390                 395                 400

Val Asp Leu Ala Lys Arg Ala Leu Trp Thr Pro Asp Gln Ile Thr Asp
                405                 410                 415

Ile Thr Ser Leu Val Arg Leu Val Tyr Ile Leu Ser Lys Gln Asn Gln
            420                 425                 430

Gln His Leu Ile Pro Gln Trp Ala Leu Arg Gln Ile Ala Asp Phe Ala
        435                 440                 445

Leu Gln Leu His Lys Thr His Leu Ala Ser Phe Leu Ser Ala Phe Ala
    450                 455                 460

Arg Gln Glu Leu Tyr Leu Met Gly Ser Leu Val His Ser Met Leu Val
465                 470                 475                 480

His Thr Thr Glu Arg Arg Glu Ile Phe Ile Val Glu Thr Gly Leu Cys
                485                 490                 495

Ser Leu Ala Glu Leu Ser His Phe Thr Gln Leu Leu Ala His Pro His
            500                 505                 510

His Glu Tyr Leu Ser Asp Leu Tyr Thr Pro Cys Ser Ser Ser Gly Arg
        515                 520                 525

Arg Asp His Ser Leu Glu Arg Leu Thr Arg Leu Phe Pro Asp Ala Thr
    530                 535                 540

Val Pro Ala Thr Val Pro Ala Ala Leu Ser Ile Leu Ser Thr Met Gln
545                 550                 555                 560

Pro Ser Thr Leu Glu Thr Phe Pro Asp Leu Phe Cys Leu Pro Leu Gly
                565                 570                 575

Glu Ser Phe Ser Ala Leu Thr Val Ser Glu His Val Ser Tyr Val Val
            580                 585                 590

Thr Asn Gln Tyr Leu Ile Lys Gly Ile Ser Tyr Pro Val Ser Thr Thr
        595                 600                 605

Val Val Gly Gln Ser Leu Ile Ile Thr Gln Thr Asp Ser Gln Thr Lys
    610                 615                 620

Cys Glu Leu Thr Arg Asn Met His Thr Thr His Ser Ile Thr Ala Ala
625                 630                 635                 640

Leu Asn Ile Ser Leu Glu Asn Cys Ala Phe Cys Gln Ser Ala Leu Leu
                645                 650                 655

Glu Tyr Asp Asp Thr Gln Gly Val Ile Asn Ile Met Tyr Met His Asp
            660                 665                 670

Ser Asp Asp Val Leu Phe Ala Leu Asp Pro Tyr Asn Glu Val Val Val
        675                 680                 685

Ser Ser Pro Arg Thr His Tyr Leu Met Leu Leu Lys Asn Gly Thr Val
    690                 695                 700

Leu Glu Val Thr Asp Val Val Val Asp Ala Thr Asp Ser Arg Leu Leu
705                 710                 715                 720

Met Met Ser Val Tyr Ala Leu Ser Ala Ile Ile Gly Ile Tyr Leu Leu
                725                 730                 735

Tyr Arg Met Leu Lys Thr Cys
            740

<210> 4
<211> 715
<212> PRT
<213> Human herpesvirus 5

<400> 4
   Met Arg Pro Gly Leu Pro Ser Tyr Leu Ile Ile Leu Ala Val Cys Leu
   1               5                   10                  15
   Phe Ser His Leu Leu Ser Ser Arg Tyr Gly Ala Glu Ala Val Ser Glu
               20                  25                  30
   Pro Leu Asp Lys Ala Phe His Leu Leu Leu Asn Thr Tyr Gly Arg Pro
           35                  40                  45
   Ile Arg Phe Leu Arg Glu Asn Thr Thr Gln Cys Thr Tyr Asn Ser Ser
       50                  55                  60
   Leu Arg Asn Ser Thr Val Val Arg Glu Asn Ala Ile Ser Phe Asn Phe
   65                  70                  75                  80
   Phe Gln Ser Tyr Asn Gln Tyr Tyr Val Phe His Met Pro Arg Cys Leu
                   85                  90                  95
   Phe Ala Gly Pro Leu Ala Glu Gln Phe Leu Asn Gln Val Asp Leu Thr
               100                 105                 110
   Glu Thr Leu Glu Arg Tyr Gln Gln Arg Leu Asn Thr Tyr Ala Leu Val
               115                 120                 125
   Ser Lys Asp Leu Ala Ser Tyr Arg Ser Phe Ser Gln Gln Leu Lys Ala
       130                 135                 140
   Gln Asp Ser Leu Gly Glu Gln Pro Thr Thr Val Pro Pro Pro Ile Asp
   145                 150                 155                 160
   Leu Ser Ile Pro His Val Trp Met Pro Pro Gln Thr Thr Pro His Gly
                   165                 170                 175
   Trp Thr Glu Ser His Thr Thr Ser Gly Leu His Arg Pro His Phe Asn
               180                 185                 190
   Gln Thr Cys Ile Leu Phe Asp Gly His Asp Leu Leu Phe Ser Thr Val
           195                 200                 205
   Thr Pro Cys Leu His Gln Gly Phe Tyr Leu Ile Asp Glu Leu Arg Tyr
       210                 215                 220
   Val Lys Ile Thr Leu Thr Glu Asp Phe Phe Val Val Thr Val Ser Ile
   225                 230                 235                 240
   Asp Asp Asp Thr Pro Met Leu Leu Ile Phe Gly His Leu Pro Arg Val
                   245                 250                 255
   Leu Phe Lys Ala Pro Tyr Gln Arg Asp Asn Phe Ile Leu Arg Gln Thr
               260                 265                 270
   Glu Lys His Glu Leu Leu Val Leu Val Lys Lys Asp Gln Leu Asn Arg
           275                 280                 285
   His Ser Tyr Leu Lys Asp Pro Asp Phe Leu Asp Ala Ala Leu Asp Phe
       290                 295                 300
   Asn Tyr Leu Asp Leu Ser Ala Leu Leu Arg Asn Ser Phe His Arg Tyr
   305                 310                 315                 320
   Ala Val Asp Val Leu Lys Ser Gly Arg Cys Gln Met Leu Asp Arg Arg
                   325                 330                 335
   Thr Val Glu Met Ala Phe Ala Tyr Ala Leu Ala Leu Phe Ala Ala Ala
               340                 345                 350
   Arg Gln Glu Glu Ala Gly Ala Gln Val Ser Val Pro Arg Ala Leu Asp
           355                 360                 365
   Arg Gln Ala Ala Leu Leu Gln Ile Gln Glu Phe Met Ile Thr Cys Leu
       370                 375                 380
   Ser Gln Thr Pro Pro Arg Thr Thr Leu Leu Leu Tyr Pro Thr Ala Val
   385                 390                 395                 400
   Asp Leu Ala Lys Arg Ala Leu Trp Thr Pro Asn Gln Ile Thr Asp Ile
                   405                 410                 415
   Thr Ser Leu Val Arg Leu Val Tyr Ile Leu Ser Lys Gln Asn Gln Gln
               420                 425                 430
   His Leu Ile Pro Gln Trp Ala Leu Arg Gln Ile Ala Asp Phe Ala Leu
           435                 440                 445
   Lys Leu His Lys Thr His Leu Ala Ser Phe Leu Ser Ala Phe Ala Arg
       450                 455                 460
   Gln Glu Leu Tyr Leu Met Gly Ser Leu Val His Ser Met Leu Val His
   465                 470                 475                 480
   Thr Thr Glu Arg Arg Glu Ile Phe Ile Val Glu Thr Gly Leu Cys Ser
                   485                 490                 495
   Leu Ala Glu Leu Ser His Phe Thr Gln Leu Leu Ala His Pro His His
               500                 505                 510
   Glu Tyr Leu Ser Asp Leu Tyr Thr Pro Cys Ser Ser Ser Gly Arg Arg
           515                 520                 525
   Asp His Ser Leu Glu Arg Leu Thr Arg Leu Phe Pro Asp Ala Thr Val
       530                 535                 540
   Pro Ala Thr Val Pro Ala Ala Leu Ser Ile Leu Ser Thr Met Gln Pro
   545                 550                 555                 560
   Ser Thr Leu Glu Thr Phe Pro Asp Leu Phe Cys Leu Pro Leu Gly Glu
                   565                 570                 575
   Ser Phe Ser Ala Leu Thr Val Ser Glu His Val Ser Tyr Ile Val Thr
               580                 585                 590
   Asn Gln Tyr Leu Ile Lys Gly Ile Ser Tyr Pro Val Ser Thr Thr Val
           595                 600                 605
   Val Gly Gln Ser Leu Ile Ile Thr Gln Thr Asp Ser Gln Thr Lys Cys
   610                 615                 620
   Glu Leu Thr Arg Asn Met His Thr Thr His Ser Ile Thr Val Ala Leu
   625                 630                 635                 640
   Asn Ile Ser Leu Glu Asn Cys Ala Phe Cys Gln Ser Ala Leu Leu Glu
                   645                 650                 655
   Tyr Asp Asp Thr Gln Gly Val Ile Asn Ile Met Tyr Met His Asp Ser
               660                 665                 670
   Asp Asp Val Leu Phe Ala Leu Asp Pro Tyr Asn Glu Val Val Val Ser
           675                 680                 685
   Ser Pro Arg Thr His Tyr Leu Met Leu Leu Lys Asn Gly Thr Val Leu
       690                 695                 700
   Glu Val Thr Asp Val Val Val Asp Ala Thr Asp
   705                 710                 715

<210> 5
<211> 692
<212> PRT
<213> Human herpesvirus 5

<400> 5
Arg Tyr Gly Ala Glu Ala Val Ser Glu Pro Leu Asp Lys Ala Phe His
1               5                   10                  15

Leu Leu Leu Asn Thr Tyr Gly Arg Pro Ile Arg Phe Leu Arg Glu Asn
            20                  25                  30

Thr Thr Gln Cys Thr Tyr Asn Ser Ser Leu Arg Asn Ser Thr Val Val
        35                  40                  45

Arg Glu Asn Ala Ile Ser Phe Asn Phe Phe Gln Ser Tyr Asn Gln Tyr
    50                  55                  60

Tyr Val Phe His Met Pro Arg Cys Leu Phe Ala Gly Pro Leu Ala Glu
65                  70                  75                  80

Gln Phe Leu Asn Gln Val Asp Leu Thr Glu Thr Leu Glu Arg Tyr Gln
                85                  90                  95

Gln Arg Leu Asn Thr Tyr Ala Leu Val Ser Lys Asp Leu Ala Ser Tyr
            100                 105                 110

Arg Ser Phe Ser Gln Gln Leu Lys Ala Gln Asp Ser Leu Gly Glu Gln
        115                 120                 125

Pro Thr Thr Val Pro Pro Pro Ile Asp Leu Ser Ile Pro His Val Trp
    130                 135                 140

Met Pro Pro Gln Thr Thr Pro His Gly Trp Thr Glu Ser His Thr Thr
145                 150                 155                 160

Ser Gly Leu His Arg Pro His Phe Asn Gln Thr Cys Ile Leu Phe Asp
                165                 170                 175

Gly His Asp Leu Leu Phe Ser Thr Val Thr Pro Cys Leu His Gln Gly
            180                 185                 190

Phe Tyr Leu Ile Asp Glu Leu Arg Tyr Val Lys Ile Thr Leu Thr Glu
        195                 200                 205

Asp Phe Phe Val Val Thr Val Ser Ile Asp Asp Asp Thr Pro Met Leu
    210                 215                 220

Leu Ile Phe Gly His Leu Pro Arg Val Leu Phe Lys Ala Pro Tyr Gln
225                 230                 235                 240

Arg Asp Asn Phe Ile Leu Arg Gln Thr Glu Lys His Glu Leu Leu Val
                245                 250                 255

Leu Val Lys Lys Asp Gln Leu Asn Arg His Ser Tyr Leu Lys Asp Pro
            260                 265                 270

Asp Phe Leu Asp Ala Ala Leu Asp Phe Asn Tyr Leu Asp Leu Ser Ala
        275                 280                 285

Leu Leu Arg Asn Ser Phe His Arg Tyr Ala Val Asp Val Leu Lys Ser
    290                 295                 300

Gly Arg Cys Gln Met Leu Asp Arg Arg Thr Val Glu Met Ala Phe Ala
305                 310                 315                 320

Tyr Ala Leu Ala Leu Phe Ala Ala Ala Arg Gln Glu Glu Ala Gly Ala
                325                 330                 335

Gln Val Ser Val Pro Arg Ala Leu Asp Arg Gln Ala Ala Leu Leu Gln
            340                 345                 350

Ile Gln Glu Phe Met Ile Thr Cys Leu Ser Gln Thr Pro Pro Arg Thr
        355                 360                 365

Thr Leu Leu Leu Tyr Pro Thr Ala Val Asp Leu Ala Lys Arg Ala Leu

370                 375                 380

Trp Thr Pro Asn Gln Ile Thr Asp Ile Thr Ser Leu Val Arg Leu Val
385                 390                 395                 400

Tyr Ile Leu Ser Lys Gln Asn Gln Gln His Leu Ile Pro Gln Trp Ala
                405                 410                 415

Leu Arg Gln Ile Ala Asp Phe Ala Leu Lys Leu His Lys Thr His Leu
            420                 425                 430

Ala Ser Phe Leu Ser Ala Phe Ala Arg Gln Glu Leu Tyr Leu Met Gly
        435                 440                 445

Ser Leu Val His Ser Met Leu Val His Thr Thr Glu Arg Arg Glu Ile
    450                 455                 460

Phe Ile Val Glu Thr Gly Leu Cys Ser Leu Ala Glu Leu Ser His Phe
465                 470                 475                 480

Thr Gln Leu Leu Ala His Pro His His Glu Tyr Leu Ser Asp Leu Tyr
                485                 490                 495

Thr Pro Cys Ser Ser Ser Gly Arg Arg Asp His Ser Leu Glu Arg Leu
            500                 505                 510

Thr Arg Leu Phe Pro Asp Ala Thr Val Pro Ala Thr Val Pro Ala Ala
        515                 520                 525

Leu Ser Ile Leu Ser Thr Met Gln Pro Ser Thr Leu Glu Thr Phe Pro
    530                 535                 540

Asp Leu Phe Cys Leu Pro Leu Gly Glu Ser Phe Ser Ala Leu Thr Val
545                 550                 555                 560

Ser Glu His Val Ser Tyr Ile Val Thr Asn Gln Tyr Leu Ile Lys Gly
                565                 570                 575

Ile Ser Tyr Pro Val Ser Thr Thr Val Val Gly Gln Ser Leu Ile Ile
            580                 585                 590

Thr Gln Thr Asp Ser Gln Thr Lys Cys Glu Leu Thr Arg Asn Met His
        595                 600                 605

Thr Thr His Ser Ile Thr Val Ala Leu Asn Ile Ser Leu Glu Asn Cys
    610                 615                 620

Ala Phe Cys Gln Ser Ala Leu Leu Glu Tyr Asp Asp Thr Gln Gly Val
625                 630                 635                 640

Ile Asn Ile Met Tyr Met His Asp Ser Asp Asp Val Leu Phe Ala Leu
                645                 650                 655

Asp Pro Tyr Asn Glu Val Val Val Ser Ser Pro Arg Thr His Tyr Leu
            660                 665                 670

Met Leu Leu Lys Asn Gly Thr Val Leu Glu Val Thr Asp Val Val Val
        675                 680                 685

Asp Ala Thr Asp
    690

<210> 6
<211> 300
<212> PRT
<213> Human herpesvirus

<400> 6
Met Lys Arg Arg Arg Arg Trp Arg Gly Trp Leu Leu Phe Leu Ala Leu
1               5                   10                  15

Cys Phe Cys Leu Leu Cys Glu Ala Val Glu Thr Asn Ala Thr Thr Val
            20                  25                  30

Thr Ser Thr Thr Ala Ala Ala Ala Thr Thr Asn Thr Thr Val Ala Thr
        35                  40                  45

Thr Gly Thr Thr Thr Thr Ser Pro Asn Val Thr Ser Thr Thr Ser Asn
    50                  55                  60

Thr Val Thr Thr Pro Thr Thr Val Ser Ser Val Ser Asn Leu Thr Ser
65                  70                  75                  80

Ser Ala Thr Ser Ile Leu Ile Ser Thr Ser Thr Val Ser Gly Thr Arg
                85                  90                  95

Asn Thr Arg Asn Asn Asn Thr Thr Thr Ile Gly Thr Asn Ala Thr Ser
            100                 105                 110

Pro Ser Ser Ser Val Ser Ile Leu Thr Thr Val Thr Pro Ala Ala Thr
        115                 120                 125

Ser Thr Thr Ser Asn Asn Gly Asp Val Thr Ser Asp Tyr Thr Pro Thr
    130                 135                 140

Phe Asp Leu Glu Asn Ile Thr Thr Thr Arg Ala Pro Thr Arg Pro Pro

145                 150                 155                 160

Ala Gln Asp Leu Cys Ser His Asn Leu Ser Ile Ile Leu Tyr Glu Glu
                165                 170                 175

Glu Ser Gln Ser Ser Val Asp Ile Ala Val Asp Glu Glu Glu Pro Glu
            180                 185                 190

Leu Glu Asp Asp Asp Glu Tyr Asp Glu Leu Trp Phe Pro Leu Tyr Phe
        195                 200                 205

Glu Ala Glu Cys Asn Leu Asn Tyr Thr Leu Gln Tyr Val Asn His Ser
    210                 215                 220

Cys Asp Tyr Ser Val Arg Gln Ser Ser Val Ser Phe Pro Pro Trp Arg
225                 230                 235                 240

Asp Ile Asp Ser Val Thr Phe Val Pro Arg Asn Leu Ser Asn Cys Ser
                245                 250                 255

Ala His Gly Leu Ala Val Ile Val Ala Gly Asn Gln Thr Trp Tyr Val
            260                 265                 270

Asn Pro Phe Ser Leu Ala His Leu Leu Asp Ala Ile Tyr Asn Val Leu
        275                 280                 285

Gly Ile Glu Asp Leu Ser Ala Asn Phe Trp Arg Gln
    290                 295                 300

<210> 7
<211> 300
<212> PRT
<213> Human herpesvirus

<400> 7
Met Lys Arg Arg Arg Arg Trp Arg Gly Trp Leu Leu Phe Leu Ala Leu
1               5                   10                  15

Cys Phe Cys Leu Leu Cys Glu Ala Val Glu Thr Asn Ala Thr Thr Val
            20                  25                  30

Thr Gly Thr Thr Ala Ala Ala Ala Thr Thr Asn Thr Thr Val Ala Thr
        35                  40                  45

Thr Gly Thr Thr Thr Thr Ser Pro Asn Val Thr Ser Thr Thr Ser Asn
    50                  55                  60

Thr Val Thr Thr Pro Thr Thr Val Ser Ser Val Ser Asn Leu Thr Ser
65                  70                  75                  80

Ser Thr Thr Ser Ile Pro Ile Ser Thr Ser Thr Val Ser Gly Thr Arg
                85                  90                  95

Asn Thr Gly Asn Asn Asn Thr Thr Thr Ile Gly Thr Asn Ala Thr Ser
            100                 105                 110

Pro Ser Pro Ser Val Ser Ile Leu Thr Thr Ala Thr Pro Ala Ala Thr
        115                 120                 125

Ser Thr Thr Ser Asn Asn Gly Asp Val Thr Ser Asp Tyr Thr Pro Thr
    130                 135                 140

Phe Asp Leu Glu Asn Ile Thr Thr Thr Arg Ala Pro Thr Arg Pro Pro
145                 150                 155                 160

Ala Gln Asp Leu Cys Ser His Asn Leu Ser Ile Ile Leu Tyr Glu Glu
                165                 170                 175

Glu Ser Gln Ser Ser Val Asp Ile Ala Val Asp Glu Glu Glu Pro Glu
            180                 185                 190

Leu Glu Asp Asp Asp Glu Tyr Asp Glu Leu Trp Phe Pro Leu Tyr Phe
        195                 200                 205

Glu Ala Glu Cys Asn Leu Asn Tyr Thr Leu Gln Tyr Val Asn His Ser
    210                 215                 220

Cys Asp Tyr Ser Val Arg Gln Ser Ser Val Ser Phe Pro Pro Trp Arg
225                 230                 235                 240

Asp Ile Asp Ser Val Thr Phe Val Pro Arg Asn Leu Ser Asn Cys Ser
                245                 250                 255

Ala His Gly Leu Ala Val Ile Val Ala Gly Asn Gln Thr Trp Tyr Val
            260                 265                 270

Asn Pro Phe Ser Leu Ala His Leu Leu Asp Ala Ile Tyr Asn Val Leu
        275                 280                 285

Gly Ile Glu Asp Leu Ser Ala Asn Phe Arg Arg Gln
    290                 295                 300

<210> 8
<211> 300
<212> PRT
<213> Human herpesvirus

<400> 8
Met Lys Arg Arg Arg Arg Trp Arg Gly Trp Leu Leu Phe Leu Ala Leu
1               5                   10                  15

Cys Phe Cys Leu Leu Cys Glu Ala Val Glu Thr Asn Ala Thr Thr Val
            20                  25                  30

Thr Ser Thr Thr Ala Ala Ala Ala Thr Thr Asn Thr Thr Val Ala Thr
        35                  40                  45

Thr Gly Thr Thr Thr Thr Ser Pro Asn Val Thr Ser Thr Thr Ser Asn
    50                  55                  60

Thr Val Ile Thr Pro Thr Thr Val Ser Ser Val Ser Asn Leu Thr Ser
65                  70                  75                  80

Ser Ala Thr Ser Ile Pro Ile Ser Thr Ser Thr Val Ser Gly Thr Arg
                85                  90                  95

Asn Thr Arg Asn Asn Asn Thr Thr Thr Ile Gly Thr Asn Val Thr Ser
            100                 105                 110

Pro Ser Pro Ser Val Ser Ile Leu Thr Thr Val Thr Pro Ala Ala Thr
        115                 120                 125

Ser Thr Thr Ser Asn Asn Gly Asp Val Thr Ser Asp Tyr Thr Pro Thr
    130                 135                 140

Phe Asp Leu Glu Asn Ile Thr Thr Thr Arg Ala Pro Thr Arg Pro Pro
145                 150                 155                 160

Ala Gln Asp Leu Cys Ser His Asn Leu Ser Ile Ile Leu Tyr Glu Glu
                165                 170                 175

Glu Ser Gln Ser Ser Val Asp Ile Ala Val Asp Glu Glu Glu Pro Glu
            180                 185                 190

Leu Glu Asp Asp Asp Glu Tyr Asp Glu Leu Trp Phe Pro Leu Tyr Phe
        195                 200                 205

Glu Ala Glu Cys Asn Leu Asn Tyr Thr Leu Gln Tyr Val Asn His Ser
    210                 215                 220

Cys Asp Tyr Ser Val Arg Gln Ser Ser Val Ser Phe Pro Pro Trp Arg
225                 230                 235                 240

Asp Ile Asp Ser Val Thr Phe Val Pro Arg Asn Leu Ser Asn Cys Ser
                245                 250                 255

Ala His Gly Leu Ala Val Ile Val Ala Gly Asn Gln Thr Trp Tyr Val
            260                 265                 270

Asn Pro Phe Ser Leu Ala His Leu Leu Asp Ala Ile Tyr Asn Val Leu
        275                 280                 285

Gly Ile Glu Asp Leu Ser Ala Asn Phe Arg Arg Gln
    290                 295                 300

<210> 9
<211> 300
<212> PRT
<213> Human herpesvirus

<400> 9
Met Lys Arg Arg Arg Arg Trp Arg Gly Trp Leu Leu Phe Leu Ala Leu
1               5                   10                  15

Cys Phe Cys Leu Leu Cys Glu Ala Val Glu Thr Asn Ala Thr Thr Val
            20                  25                  30

Thr Ser Thr Thr Ala Ala Ala Ala Thr Thr Asn Thr Thr Val Ala Thr
        35                  40                  45

Thr Gly Thr Thr Thr Thr Ser Pro Asn Val Thr Ser Thr Thr Ser Asn
    50                  55                  60

Thr Val Thr Thr Pro Thr Thr Val Ser Ser Val Ser Asn Leu Thr Ser
65                  70                  75                  80

Ser Ala Thr Ser Ile Pro Ile Ser Thr Ser Thr Val Ser Glu Thr Arg
                85                  90                  95

Asn Thr Arg Asn Asn Asn Thr Thr Thr Ile Gly Thr Asn Ala Thr Ser
            100                 105                 110

Pro Ser Pro Ser Val Ser Ile Leu Thr Thr Val Thr Pro Ala Ala Thr
        115                 120                 125

Ser Thr Ile Ser Val Asp Gly Val Val Thr Ala Ser Asp Tyr Thr Pro
    130                 135                 140

Thr Phe Asp Asp Leu Glu Asn Ile Thr Thr Thr Arg Ala Pro Thr Arg
145                 150                 155                 160

Pro Pro Ala Gln Asp Leu Cys Ser His Asn Leu Ser Ile Ile Leu Tyr
                165                 170                 175

Glu Glu Glu Ser Gln Ser Ser Val Asp Ile Ala Val Asp Glu Glu Glu
                180                 185                 190

Pro Glu Leu Glu Asp Asp Asp Glu Tyr Asp Glu Leu Trp Phe Pro Leu
        195                 200                 205

Tyr Phe Glu Ala Glu Cys Asn Leu Asn Tyr Thr Leu Gln Tyr Val Asn
    210                 215                 220

His Ser Cys Asp Tyr Ser Val Arg Gln Ser Ser Val Ser Phe Pro Pro
225                 230                 235                 240

Trp Arg Asp Ile Asp Ser Val Thr Phe Val Pro Arg Asn Leu Ser Asn
                245                 250                 255

Cys Ser Ala His Gly Leu Ala Val Ile Val Ala Gly Asn Gln Thr Trp
                260                 265                 270

Tyr Val Asn Pro Phe Ser Leu Ala His Leu Leu Asp Ala Ile Tyr Asn
        275                 280                 285

Val Leu Gly Ile Glu Asp Leu Ser Ala Asn Phe Arg
    290                 295                 300

<210> 10
<211> 300
<212> PRT
<213> Human herpesvirus

<400> 10
Met Lys Arg Arg Arg Arg Trp Arg Gly Trp Leu Leu Phe Pro Ala Leu
1               5                   10                  15

Cys Phe Cys Leu Leu Cys Glu Ala Val Glu Thr Asn Ala Thr Thr Val
            20                  25                  30

Thr Ser Thr Thr Ala Ala Ala Ala Thr Thr Asn Thr Thr Val Ala Thr
        35                  40                  45

Thr Gly Thr Thr Thr Thr Ser Pro Asn Val Thr Ser Thr Thr Ser Asn
    50                  55                  60

Thr Val Thr Thr Pro Thr Thr Val Ser Ser Val Ser Asn Leu Thr Ser
65                  70                  75                  80

Ser Thr Thr Ser Ile Pro Ile Ser Thr Ser Thr Val Ser Gly Thr Arg
                85                  90                  95

Asn Thr Gly Asn Asn Asn Thr Thr Thr Ile Gly Thr Asn Ala Thr Ser
            100                 105                 110

Pro Ser Pro Ser Val Ser Ile Leu Thr Thr Val Thr Pro Ala Ala Thr
        115                 120                 125

Ser Thr Ile Ser Val Asp Gly Val Val Thr Ala Ser Asp Tyr Thr Pro
    130                 135                 140

Thr Phe Asp Asp Leu Glu Asn Ile Thr Thr Thr Arg Ala Pro Thr Arg
145                 150                 155                 160

Pro Pro Ala Gln Asp Leu Cys Ser His Asn Leu Ser Ile Ile Leu Tyr
                165                 170                 175

Glu Glu Glu Ser Gln Ser Ser Val Asp Ile Ala Val Asp Glu Glu Glu
            180                 185                 190

Pro Glu Leu Glu Asp Asp Asp Glu Tyr Asp Glu Leu Trp Phe Pro Leu
        195                 200                 205

Tyr Phe Glu Ala Glu Cys Asn Arg Asn Tyr Thr Leu His Val Asn His
    210                 215                 220

Ser Cys Asp Tyr Ser Val Arg Gln Ser Ser Val Ser Phe Pro Pro Trp
225                 230                 235                 240

Arg Asp Ile Asp Ser Val Thr Phe Val Pro Arg Asn Leu Ser Asn Cys
                245                 250                 255

Ser Ala His Gly Leu Ala Val Ile Val Ala Gly Asn Gln Thr Trp Tyr
            260                 265                 270

Val Asn Pro Phe Ser Leu Ala His Leu Leu Asp Ala Ile Tyr Asn Val
        275                 280                 285

Leu Gly Ile Glu Asp Leu Ser Ala Asn Phe Arg Arg
    290                 295                 300

<210> 11
<211> 300
<212> PRT
<213> Human herpesvirus

<400> 11
Met Lys Arg Arg Arg Arg Trp Arg Gly Trp Leu Leu Phe Leu Ala Leu
1               5                   10                  15

Cys Phe Cys Leu Leu Cys Glu Ala Val Glu Thr Asn Ala Thr Thr Val

20                  25                  30

Thr Ser Thr Thr Ala Ala Ala Ala Thr Thr Asn Thr Thr Val Ala Thr
        35                  40                  45

Thr Gly Thr Thr Thr Thr Ser Pro Asn Val Thr Ser Thr Thr Ser Asn
    50                  55                  60

Thr Val Ile Thr Pro Thr Thr Val Ser Ser Val Ser Asn Leu Thr Ser
65                  70                  75                  80

Ser Ala Thr Ser Ile Pro Ile Ser Thr Ser Thr Val Ser Gly Thr Arg
                85                  90                  95

Asn Thr Arg Asn Asn Asn Thr Thr Thr Ile Gly Thr Asn Val Thr Ser
            100                 105                 110

Pro Ser Pro Ser Val Ser Ile Leu Thr Thr Val Thr Pro Ala Ala Thr
        115                 120                 125

Ser Thr Thr Ser Asn Asn Gly Asp Val Thr Ser Asp Tyr Thr Pro Thr
    130                 135                 140

Phe Asp Leu Glu Asn Ile Thr Thr Thr Arg Ala Pro Thr Arg Pro Pro
145                 150                 155                 160

Ala Gln Asp Leu Cys Ser His Asn Leu Ser Ile Ile Leu Tyr Glu Glu
                165                 170                 175

Glu Ser Gln Ser Ser Val Asp Ile Ala Val Asp Glu Glu Glu Pro Glu
            180                 185                 190

Leu Glu Asp Asp Asp Glu Tyr Asp Glu Leu Trp Phe Pro Leu Tyr Phe
        195                 200                 205

Glu Ala Glu Cys Asn Leu Asn Tyr Thr Leu Gln Tyr Val Asn His Ser
    210                 215                 220

Cys Asp Tyr Ser Val Arg Gln Ser Ser Val Ser Phe Pro Pro Trp Arg
225                 230                 235                 240

Asp Ile Asp Ser Val Thr Phe Val Pro Arg Asn Leu Ser Asn Cys Ser
                245                 250                 255

Ala His Gly Leu Ala Val Ile Val Ala Gly Asn Gln Thr Trp Tyr Val
            260                 265                 270

Asn Pro Phe Ser Leu Ala His Leu Leu Asp Ala Ile Tyr Asn Val Leu
        275                 280                 285

Gly Ile Glu Asp Leu Ser Ala Asn Phe Arg Arg Gln
    290                 295                 300

<210> 12
<211> 300
<212> PRT
<213> Human herpesvirus

<400> 12
Met Lys Arg Arg Arg Arg Trp Arg Gly Trp Leu Leu Phe Leu Ala Leu
1               5                   10                  15

Cys Phe Cys Leu Leu Cys Glu Ala Val Glu Thr Asn Ala Thr Thr Val
            20                  25                  30

Thr Ser Thr Thr Ala Ala Ala Ala Thr Thr Asn Thr Thr Val Ala Thr
        35                  40                  45

Thr Gly Thr Thr Thr Thr Ser Pro Asn Val Thr Ser Thr Thr Ser Asn
    50                  55                  60

Thr Val Thr Thr Pro Thr Thr Val Ser Ser Val Ser Asn Leu Thr Ser
65                  70                  75                  80

Ser Ala Thr Ser Ile Pro Ile Ser Thr Ser Thr Val Ser Glu Thr Arg
                85                  90                  95

Asn Thr Arg Asn Asn Asn Thr Thr Thr Ile Gly Thr Asn Ala Thr Ser
            100                 105                 110

Pro Ser Pro Ser Val Ser Ile Leu Thr Thr Val Thr Pro Ala Ala Thr
        115                 120                 125

Ser Thr Thr Ser Asn Asn Gly Asp Val Thr Ser Asp Tyr Thr Pro Thr
    130                 135                 140

Phe Asp Leu Glu Asn Ile Thr Thr Thr Arg Ala Pro Thr Arg Pro Pro
145                 150                 155                 160

Ala Gln Asp Leu Cys Ser His Asn Leu Ser Ile Ile Leu Tyr Glu Glu
                165                 170                 175

Glu Ser Gln Ser Ser Val Asp Ile Ala Val Asp Glu Glu Glu Pro Glu
            180                 185                 190

Leu Glu Asp Asp Asp Glu Tyr Asp Glu Leu Trp Phe Pro Leu Tyr Phe

195                 200                 205

Glu Ala Glu Cys Asn Leu Asn Tyr Thr Leu Gln Tyr Val Asn His Ser
    210                 215                 220

Cys Asp Tyr Ser Val Arg Gln Ser Ser Val Ser Phe Pro Pro Trp Arg
225                 230                 235                 240

Asp Ile Asp Ser Val Thr Phe Val Pro Arg Asn Leu Ser Asn Cys Ser
                245                 250                 255

Ala His Gly Leu Ala Val Ile Val Ala Gly Asn Gln Thr Trp Tyr Val
            260                 265                 270

Asn Pro Phe Ser Leu Ala His Leu Leu Asp Ala Ile Tyr Asn Val Leu
        275                 280                 285

Gly Ile Glu Asp Leu Ser Ala Asn Phe Arg Arg Gln
    290                 295                 300

<210> 13
<211> 300
<212> PRT
<213> Human herpesvirus

<400> 13
Met Lys Arg Arg Arg Arg Trp Arg Gly Trp Leu Leu Phe Leu Ala Leu
1               5                   10                  15

Cys Phe Cys Leu Leu Cys Glu Ala Val Glu Thr Asn Thr Thr Thr Val
            20                  25                  30

Thr Ser Thr Thr Ala Ala Ala Ala Thr Thr Asn Thr Thr Val Ala Thr
        35                  40                  45

Thr Gly Thr Thr Thr Thr Ser Pro Asn Val Thr Ser Thr Thr Ser Asn
    50                  55                  60

Thr Val Thr Thr Pro Thr Thr Val Ser Ser Val Ser Asn Leu Thr Ser
65                  70                  75                  80

Ser Thr Thr Ser Ile Pro Ile Ser Thr Ser Thr Val Ser Gly Thr Arg
                85                  90                  95

Asn Thr Gly Asn Asn Asn Thr Thr Thr Ile Gly Thr Asn Ala Thr Ser
            100                 105                 110

Pro Ser Pro Ser Val Ser Ile Leu Thr Thr Val Thr Pro Ala Ala Thr
        115                 120                 125

Ser Thr Thr Ser Asn Asn Gly Asp Val Thr Ser Asp Tyr Thr Pro Thr
    130                 135                 140

Phe Asp Leu Glu Asn Ile Thr Thr Thr Arg Ala Pro Thr Arg Pro Pro
145                 150                 155                 160

Ala Gln Asp Leu Cys Ser His Asn Leu Ser Ile Ile Leu Tyr Glu Glu
                165                 170                 175

Glu Ser Gln Ser Ser Val Asp Ile Thr Val Gly Glu Glu Glu Ser Glu
            180                 185                 190

Ser Glu Glu Asp Asp Asp Glu Glu Tyr Asp Glu Leu Trp Phe Pro Leu
        195                 200                 205

Tyr Phe Glu Ala Glu Cys Asn Leu Asn Tyr Thr Leu Gln Tyr Val Asn
    210                 215                 220

His Ser Cys Asp Tyr Ser Val Arg Gln Ser Ser Val Ser Phe Pro Pro
225                 230                 235                 240

Trp Arg Asp Ile Asp Ser Val Thr Phe Val Pro Arg Asn Leu Ser Asn
                245                 250                 255

Cys Ser Ala His Gly Leu Ala Val Ile Val Ala Gly Asn Gln Thr Trp
            260                 265                 270

Tyr Val Asn Pro Phe Ser Leu Ala His Leu Leu Asp Ala Ile Tyr Asn
        275                 280                 285

Val Leu Gly Ile Glu Asp Leu Ser Ala Asn Phe Arg
    290                 295                 300

<210> 14
<211> 300
<212> PRT
<213> Human herpesvirus

<400> 14
Met Lys Arg Arg Arg Arg Trp Arg Gly Trp Leu Leu Phe Leu Ala Leu
1               5                   10                  15

Cys Phe Cys Leu Leu Cys Glu Ala Val Glu Thr Asn Met Thr Thr Val
            20                  25                  30

Thr Ser Thr Thr Ala Ala Ala Ala Thr Thr Asn Thr Thr Val Ala Thr
        35                  40                  45

Thr Gly Thr Thr Thr Thr Ser Pro Asn Val Thr Ser Thr Thr Ser Asn
    50                  55                  60

Thr Ile Thr Thr Pro Thr Thr Val Ser Ser Val Ser Asn Leu Thr Ser
65                  70                  75                  80

Ser Thr Thr Ser Ile Pro Ile Ser Thr Ser Thr Val Ser Gly Thr Arg
                85                  90                  95

Asn Thr Gly Asn Asn Asn Thr Thr Thr Ile Gly Thr Asn Ala Thr Ser
            100                 105                 110

Pro Ser Pro Ser Val Ser Ile Leu Thr Thr Val Thr Pro Ala Ala Thr
        115                 120                 125

Ser Thr Thr Ser Asn Asn Gly Asp Val Thr Ser Asp Tyr Thr Pro Thr
    130                 135                 140

Phe Asp Leu Glu Asn Ile Thr Thr Thr Arg Ala Pro Thr Arg Pro Pro
145                 150                 155                 160

Ala Gln Asp Leu Cys Ser His Asn Leu Ser Ile Ile Leu Tyr Glu Glu
                165                 170                 175

Glu Ser Gln Ser Ser Val Asp Ile Thr Val Gly Glu Glu Glu Ser Glu
            180                 185                 190

Ser Glu Glu Asp Asp Asp Glu Glu Tyr Asp Glu Leu Trp Phe Pro Leu
        195                 200                 205

Tyr Phe Glu Ala Glu Cys Asn Leu Asn Tyr Thr Leu Gln Tyr Val Asn
    210                 215                 220

His Ser Cys Asp Tyr Ser Val Arg Gln Ser Ser Val Ser Phe Pro Pro
225                 230                 235                 240

Trp Arg Asp Ile Asp Ser Val Thr Phe Val Pro Arg Asn Leu Ser Asn
                245                 250                 255

Cys Ser Ala His Gly Leu Ala Val Ile Val Ala Gly Asn Gln Thr Trp
            260                 265                 270

Tyr Val Asn Pro Phe Ser Leu Ala His Leu Leu Asp Ala Ile Tyr Asn
        275                 280                 285

Val Leu Gly Ile Glu Asp Leu Ser Ala Asn Phe Arg
    290                 295                 300

<210> 15
<211> 300
<212> PRT
<213> Human herpesvirus

<400> 15
Met Lys Arg Arg Arg Arg Trp Arg Gly Trp Leu Leu Phe Leu Ala Leu
1               5                   10                  15

Cys Phe Cys Leu Leu Cys Glu Ala Val Glu Thr Asn Ala Thr Thr Val
            20                  25                  30

Thr Ser Thr Thr Ala Ala Ala Ala Thr Thr Asn Thr Thr Val Ala Thr
        35                  40                  45

Thr Gly Thr Thr Thr Thr Ser Pro Asn Val Thr Ser Thr Thr Ser Asn
    50                  55                  60

Thr Val Thr Thr Pro Thr Thr Val Ser Ser Val Ser Asn Leu Thr Ser
65                  70                  75                  80

Asn Thr Thr Ser Ile Pro Ile Ser Thr Ser Thr Val Ser Gly Thr Lys
                85                  90                  95

Ser Thr Gly Asn Asn Asn Thr Thr Thr Ile Gly Thr Asn Ala Thr Ser
            100                 105                 110

Pro Ser Pro Ser Val Ser Ile Leu Thr Thr Val Thr Pro Ala Ala Thr
        115                 120                 125

Ser Thr Ile Ser Val Asp Gly Val Val Thr Thr Ser Asp Tyr Thr Pro
    130                 135                 140

Thr Phe Asp Asp Leu Glu Asn Ile Thr Thr Thr Arg Ala Pro Thr Arg
145                 150                 155                 160

Pro Pro Ala Gln Asp Leu Cys Ser His Asn Leu Ser Ile Ile Leu Tyr
                165                 170                 175

Glu Glu Glu Ser Gln Ser Ser Val Asp Ile Ala Val Asp Glu Glu Glu
            180                 185                 190

Pro Glu Leu Glu Asp Asp Asp Glu Tyr Asp Glu Leu Trp Phe Pro Leu
        195                 200                 205

Tyr Phe Glu Ala Glu Cys Asn Leu Asn Tyr Thr Leu Gln Tyr Val Asn
    210                 215                 220

His Ser Cys Asp Tyr Ser Val Arg Gln Ser Ser Val Ser Phe Pro Pro
225                 230                 235                 240

Trp Arg Asp Ile Asp Ser Val Thr Phe Val Pro Arg Asn Leu Ser Asn
                245                 250                 255

Cys Ser Ala His Gly Leu Ala Val Ile Val Ala Gly Asn Gln Thr Trp
            260                 265                 270

Tyr Val Asn Pro Phe Ser Leu Ala His Leu Leu Asp Ala Ile Tyr Asn
        275                 280                 285

Val Leu Gly Ile Glu Asp Leu Ser Ala Asn Phe Arg
    290                 295                 300

<210> 16
<211> 300
<212> PRT
<213> Human herpesvirus

<400> 16
Met Lys Arg Arg Arg Arg Trp Arg Gly Trp Leu Leu Phe Leu Ala Leu
1               5                   10                  15

Cys Phe Cys Leu Leu Arg Glu Ala Val Glu Thr Asn Thr Thr Thr Val
            20                  25                  30

Thr Ser Thr Thr Ala Ala Ala Ala Thr Thr Asn Thr Thr Val Ala Thr
        35                  40                  45

Thr Gly Thr Thr Thr Thr Ser Pro Asn Val Thr Ser Thr Thr Ser Asn
    50                  55                  60

Thr Val Thr Thr Pro Thr Thr Val Ser Ser Val Ser Asn Leu Thr Ser
65                  70                  75                  80

Ser Ala Thr Ser Ile Pro Ile Ser Thr Ser Thr Val Ser Gly Ile Arg
                85                  90                  95

Asn Thr Gly Asn Asn Asn Thr Thr Thr Ile Gly Thr Asn Ala Thr Ser
            100                 105                 110

Pro Ser Pro Ser Val Ser Ile Leu Thr Thr Val Thr Pro Ala Ala Thr
        115                 120                 125

Ser Thr Thr Ser Asn Asn Gly Asp Val Thr Ser Asp Tyr Thr Pro Thr
    130                 135                 140

Phe Asp Leu Glu Asn Ile Thr Thr Thr Arg Ala Pro Thr Arg Pro Pro
145                 150                 155                 160

Ala Gln Asp Leu Cys Ser His Asn Leu Ser Ile Ile Leu Tyr Glu Glu
                165                 170                 175

Glu Ser Gln Ser Ser Val Asp Ile Thr Val Gly Glu Glu Glu Ser Glu
            180                 185                 190

Ser Glu Glu Asp Asp Asp Glu Glu Tyr Asp Glu Leu Trp Phe Pro Leu
        195                 200                 205

Tyr Phe Glu Ala Glu Cys Asn Leu Asn Tyr Thr Leu Gln Tyr Val Asn
    210                 215                 220

His Ser Cys Asp Tyr Ser Val Arg Gln Ser Ser Val Ser Phe Pro Pro
225                 230                 235                 240

Trp Arg Asp Ile Asp Ser Val Thr Phe Val Pro Arg Asn Leu Ser Asn
                245                 250                 255

Cys Ser Ala His Gly Leu Ala Val Ile Val Ala Gly Asn Gln Thr Trp
            260                 265                 270

Tyr Val Asn Pro Phe Ser Leu Ala His Leu Leu Asp Ala Ile Tyr Asn
        275                 280                 285

Val Leu Gly Ile Glu Asp Leu Ser Ala Asn Phe Arg
    290                 295                 300

<210> 17
<211> 300
<212> PRT
<213> Human herpesvirus

<400> 17
Met Lys Arg Arg Arg Arg Trp Arg Gly Trp Leu Leu Phe Leu Ala Leu
1               5                   10                  15

Cys Phe Cys Leu Leu Arg Glu Ala Val Glu Thr Asn Thr Thr Thr Val
            20                  25                  30

Thr Ser Thr Thr Ala Ala Ala Ala Thr Thr Asn Thr Thr Val Ala Thr
        35                  40                  45

Thr Gly Thr Thr Thr Thr Ser Pro Asn Val Thr Ser Thr Thr Ser Asn
    50                  55                  60

Thr Val Thr Thr Pro Thr Thr Val Ser Ser Val Ser Asn Leu Thr Ser

65                  70                  75                  80

Ser Ala Thr Ser Ile Pro Ile Ser Thr Ser Thr Val Ser Gly Ile Arg
                85                  90                  95

Asn Thr Gly Asn Asn Asn Thr Thr Thr Ile Gly Thr Asn Ala Thr Ser
            100                 105                 110

Pro Ser Pro Ser Val Ser Ile Leu Thr Thr Val Thr Pro Ala Ala Thr
        115                 120                 125

Ser Thr Thr Ser Asn Asn Gly Asp Val Thr Ser Asp Tyr Thr Pro Thr
    130                 135                 140

Phe Asp Leu Glu Asn Ile Thr Thr Thr Arg Ala Pro Thr Arg Pro Pro
145                 150                 155                 160

Ala Gln Asp Leu Cys Ser His Asn Leu Ser Ile Ile Leu Tyr Glu Glu
                165                 170                 175

Glu Ser Gln Ser Ser Val Asp Ile Thr Val Gly Glu Glu Glu Ser Glu
            180                 185                 190

Leu Glu Glu Asp Asp Asp Glu Glu Tyr Asp Glu Leu Trp Phe Pro Leu
        195                 200                 205

Tyr Phe Glu Ala Glu Cys Asn Arg Asn Tyr Thr Leu His Val Asn His
    210                 215                 220

Ser Cys Asp Tyr Ser Val Arg Gln Ser Ser Val Ser Phe Pro Pro Trp
225                 230                 235                 240

Arg Asp Ile Asp Ser Val Thr Phe Val Pro Arg Asn Leu Ser Asn Cys
                245                 250                 255

Ser Ala His Gly Leu Ala Val Ile Val Ala Gly Asn Gln Thr Trp Tyr
            260                 265                 270

Val Asn Pro Phe Ser Leu Ala His Leu Leu Asp Ala Ile Tyr Asn Val
        275                 280                 285

Leu Gly Ile Glu Asp Leu Ser Ala Asn Phe Arg Arg
    290                 295                 300

<210> 18
<211> 6
<212> PRT
<213> Artificial Sequence

<220>
<221> source
<223> /note="Description of Artificial Sequence: Synthetic
      6xHis tag"

<400> 18
His His His His His His
1               5


<210> 19
<211> 8
<212> PRT
<213> Artificial Sequence

<220>
<221> source
<223> /note="Description of Artificial Sequence: Synthetic
      peptide"

<400> 19
Asp Tyr Lys Asp Asp Asp Asp Lys
1               5


<210> 20
<211> 9
<212> PRT
<213> Artificial Sequence

<220>
<221> source
<223> /note="Description of Artificial Sequence: Synthetic
      peptide"

<400> 20
Ala Trp Arg His Pro Gln Phe Gly Gly
1               5


<210> 21
<211> 8
<212> PRT
<213> Artificial Sequence

<220>
<221> source
<223> /note="Description of Artificial Sequence: Synthetic
      peptide"

<400> 21
Trp Ser His Pro Gln Phe Glu Lys
1               5


<210> 22
<211> 74
<212> DNA
<213> Artificial Sequence

<220>
<221> source

<223> /note="Description of Artificial Sequence: Synthetic
      primer"

<400> 22
ttcggcgcca actggctcct taccgtcaca ctctcatcgt gccgcagact gattacaagg      60

atgacgacga taag                                                        74


<210> 23
<211> 123
<212> DNA
<213> Artificial Sequence

<220>
<221> source
<223> /note="Description of Artificial Sequence: Synthetic
      primer"

<400> 23
tatcaccggt ccaggtgaga aagagaagcc gcaatccggg cggcggcaca tcacttatcg      60

tcgtcatcct tgtaatcagt ctgcggcacg atgagcaacc aaattaaacc aattctgatt     120

tag                                                                   123


<210> 24
<211> 939
<212> DNA
<213> Human herpesvirus

<400> 24
atgaagcggc ggcggcgatg gcggggctgg ttgcttttcc tggccctgtg cttttgctta      60

ctgtgtgaag cggtggaaac caacgcgacc accgttacca gtaccaccgc tgccgccgcc     120

acgacaaaca ctaccgtcgc caccaccggt accactacta cctcccctaa cgtcacttca     180

accacgagta acaccgtcat cactcccacc acggtttcct cggtcagcaa tctgacatcc     240

agcgccacgt cgattcccat ctcaacgtca acggtttctg gaacaagaaa cacaaggaat     300

aataatacca caaccatcgg tacgaacgtt acttccccct ccccttctgt atccatactt     360

accaccgtga caccggccgc gacttctacc acctccaaca acggggatgt aacatccgac     420

tacactccaa cttttgacct ggaaaacatt accaccaccc gcgctcccac gcgtcctccc     480

gcccaggacc tttgtagcca taacctgtca atcatcctgt acgaagagga atctcagagc     540

agcgtagaca ttgcggtgga tgaagaagag ccagaactgg aggacgacga cgagtacgac     600

gaactgtggt tccccctcta cttcgaggct gagtgcaacc taaattacac gctacaatac     660

gtcaatcaca gttgtgatta cagcgtgcgc cagtcgtctg tctcattccc cccgtggcgc     720

gacatcgact cagttacctt cgtacccagg aacctctcca actgtagcgc ccacggtctg     780

gccgtcatcg tcgcgggtaa ccaaacctgg tacgtgaatc cgtttagcct ggctcacctg     840

ctggatgcaa tatataacgt tttagggatc gaagacctga gcgccaactt tcggcgccaa     900

ctggctcctt accgtcacac tctcatcgtg ccgcagact                                939

<210> 25
<211> 313
<212> PRT
<213> Human herpesvirus

<400> 25
Met Lys Arg Arg Arg Arg Trp Arg Gly Trp Leu Leu Phe Leu Ala Leu
1               5                   10                  15

Cys Phe Cys Leu Leu Cys Glu Ala Val Glu Thr Asn Ala Thr Thr Val
            20                  25                  30

Thr Ser Thr Thr Ala Ala Ala Ala Thr Thr Asn Thr Thr Val Ala Thr
        35                  40                  45

Thr Gly Thr Thr Thr Thr Ser Pro Asn Val Thr Ser Thr Thr Ser Asn
    50                  55                  60

Thr Val Ile Thr Pro Thr Thr Val Ser Ser Val Ser Asn Leu Thr Ser
65                  70                  75                  80

Ser Ala Thr Ser Ile Pro Ile Ser Thr Ser Thr Val Ser Gly Thr Arg
                85                  90                  95

Asn Thr Arg Asn Asn Asn Thr Thr Thr Ile Gly Thr Asn Val Thr Ser
            100                 105                 110

Pro Ser Pro Ser Val Ser Ile Leu Thr Thr Val Thr Pro Ala Ala Thr
        115                 120                 125

Ser Thr Thr Ser Asn Asn Gly Asp Val Thr Ser Asp Tyr Thr Pro Thr
    130                 135                 140

Phe Asp Leu Glu Asn Ile Thr Thr Thr Arg Ala Pro Thr Arg Pro Pro
145                 150                 155                 160

Ala Gln Asp Leu Cys Ser His Asn Leu Ser Ile Ile Leu Tyr Glu Glu
                165                 170                 175

Glu Ser Gln Ser Ser Val Asp Ile Ala Val Asp Glu Glu Glu Pro Glu
            180                 185                 190

Leu Glu Asp Asp Asp Glu Tyr Asp Glu Leu Trp Phe Pro Leu Tyr Phe
        195                 200                 205

Glu Ala Glu Cys Asn Leu Asn Tyr Thr Leu Gln Tyr Val Asn His Ser
    210                 215                 220

Cys Asp Tyr Ser Val Arg Gln Ser Ser Val Ser Phe Pro Pro Trp Arg
225                 230                 235                 240

Asp Ile Asp Ser Val Thr Phe Val Pro Arg Asn Leu Ser Asn Cys Ser
                245                 250                 255

Ala His Gly Leu Ala Val Ile Val Ala Gly Asn Gln Thr Trp Tyr Val
            260                 265                 270

Asn Pro Phe Ser Leu Ala His Leu Leu Asp Ala Ile Tyr Asn Val Leu
        275                 280                 285

Gly Ile Glu Asp Leu Ser Ala Asn Phe Arg Arg Gln Leu Ala Pro Tyr
    290                 295                 300

Arg His Thr Leu Ile Val Pro Gln Thr
305                 310

<210> 26
<211> 939
<212> DNA
<213> Artificial Sequence

<220>
<221> source
<223> /note="Description of Artificial Sequence: Synthetic
      polynucleotide"

<400> 26
atgaagcggc ggagaagatg gcggggctgg ctgctgttcc tggccctgtg cttctgtctg      60

ctgtgcgagg ccgtggagac aaacgccacc accgtgaccg gaacaacagc cgccgctgcc     120

accaccaata ccactgtcgc caccaccggc accaccacca cctcccccaa cgtgaccagc     180

accacaagca acaccgtgac cacccctacc accgtgtcca gcgtgtccaa cctgacctcc     240

agcacaacct ccatccccat cagcaccagc accgtgtccg gcacccggaa caccggcaac     300

aacaatacca ccaccatcgg gactaacgct acctctccca gcccttccgt gagcatcctg     360

accacagcca ccccagccgc tacctccaca accagcaaca acggcgacgt gacctccgac     420

tacaccccca ccttcgacct ggaaaacatc accaccacaa gagcccctac cagaccccct     480

gcccaggatc tgtgcagcca caacctgagc atcatcctgt acgaggaaga gtcccagagc     540

agcgtggata tcgccgtgga cgaggaagaa cccgagctgg aagatgacga cgagtacgac     600

gagctgtggt tccccctgta cttcgaggcc gagtgcaacc tgaactacac cctgcagtac     660

gtgaaccaca gctgcgacta cagcgtgcgg cagtcctccg tgagcttccc cccctggcgg     720

gacatcgaca gcgtgacctt cgtgccccgg aacctgagca attgcagcgc ccacggcctg     780

gctgtgatcg tggccggcaa ccagacttgg tacgtgaatc ccttcagcct ggcccacctg 840

ctggacgcca tctacaacgt gctgggcatc gaggacctga gcgccaactt cagacggcag 900

ctggccccct acagacacac cctgatcgtg ccccagacc 939

<210> 27
<211> 313
<212> PRT
<213> Artificial Sequence

<220>
<221> source
<223> /note="Description of Artificial Sequence: Synthetic
      polypeptide"

<400> 27
Met Lys Arg Arg Arg Arg Trp Arg Gly Trp Leu Leu Phe Leu Ala Leu
1               5                   10                  15

Cys Phe Cys Leu Leu Cys Glu Ala Val Glu Thr Asn Ala Thr Thr Val
            20                  25                  30

Thr Gly Thr Thr Ala Ala Ala Ala Thr Thr Asn Thr Thr Val Ala Thr
        35                  40                  45

Thr Gly Thr Thr Thr Thr Ser Pro Asn Val Thr Ser Thr Thr Ser Asn
    50                  55                  60

Thr Val Thr Thr Pro Thr Thr Val Ser Ser Val Ser Asn Leu Thr Ser
65                  70                  75                  80

Ser Thr Thr Ser Ile Pro Ile Ser Thr Ser Thr Val Ser Gly Thr Arg
                85                  90                  95

Asn Thr Gly Asn Asn Asn Thr Thr Thr Ile Gly Thr Asn Ala Thr Ser
            100                 105                 110

Pro Ser Pro Ser Val Ser Ile Leu Thr Thr Ala Thr Pro Ala Ala Thr
        115                 120                 125

Ser Thr Thr Ser Asn Asn Gly Asp Val Thr Ser Asp Tyr Thr Pro Thr
    130                 135                 140

Phe Asp Leu Glu Asn Ile Thr Thr Thr Arg Ala Pro Thr Arg Pro Pro
145                 150                 155                 160

Ala Gln Asp Leu Cys Ser His Asn Leu Ser Ile Ile Leu Tyr Glu Glu
                165                 170                 175

Glu Ser Gln Ser Ser Val Asp Ile Ala Val Asp Glu Glu Glu Pro Glu

180                 185                 190

Leu Glu Asp Asp Asp Glu Tyr Asp Glu Leu Trp Phe Pro Leu Tyr Phe
        195                 200                 205

Glu Ala Glu Cys Asn Leu Asn Tyr Thr Leu Gln Tyr Val Asn His Ser
    210                 215                 220

Cys Asp Tyr Ser Val Arg Gln Ser Ser Val Ser Phe Pro Pro Trp Arg
225                 230                 235                 240

Asp Ile Asp Ser Val Thr Phe Val Pro Arg Asn Leu Ser Asn Cys Ser
                245                 250                 255

Ala His Gly Leu Ala Val Ile Val Ala Gly Asn Gln Thr Trp Tyr Val
            260                 265                 270

Asn Pro Phe Ser Leu Ala His Leu Leu Asp Ala Ile Tyr Asn Val Leu
        275                 280                 285

Gly Ile Glu Asp Leu Ser Ala Asn Phe Arg Arg Gln Leu Ala Pro Tyr
    290                 295                 300

Arg His Thr Leu Ile Val Pro Gln Thr
305                 310